# FASCICULE DE BREVET EUROPEEN

(11) **EP 1 325 729 B1**
(45) Date de publication et mention de la délivrance du brevet: **19.04.2006**
(21) Numéro de dépôt: 02293007.7
(22) Date de dépôt: 05.12.2002
(51) Int. Cl.: A61K 8/06, A61K 8/67, A61K 8/81, A61K 8/89, A61Q 1/02, A61Q 1/12, A61Q 17/04, A61Q 19/00

(54) **Composition contenant un copolymère siliconé et soit un polymère d'un monomère à insaturation éthylénique et à groupement sulfonique, soit une poudre organique; ses utilisations, notamment cosmétiques**
Ein Silikon-Copolymer und entweder ein Polymer aus einem ethylenisch ungesättigten Monomer mit Sulfongruppen oder ein organisches Pulver enthaltende Zusammensetzung; deren Verwendungen, insbesondere in der Kosmetik
Composition containing a silicone copolymer and either a polymer from an ethylenically unsaturated monomer with sulfonic groups or an organic powder; uses thereof, especially in cosmetics

(30) Priorité: 04.01.2002 FR 0200097; 04.01.2002 FR 0200099; 04.01.2002 FR 0200096; 04.01.2002 FR 0200095
(43) Date de publication de la demande: 09.07.2003
(73) Titulaire: L'OREAL, 75008 Paris (FR)
(72) Inventeur: Lennon, Paula, 69003 Lyon (FR)
(74) Mandataire: Rasson, Catherine

(56) Documents cités:
- EP-A- 0 832 637
- EP-A- 1 013 700
- EP-A- 1 097 703
- US-A- 5 412 004
- US-A- 5 965 115
- US-A1- 2001 041 768

## Description

La présente demande se rapporte à une composition comprenant une phase aqueuse comprenant une dispersion aqueuse de particules d'un copolymère siliconé bloc substantiellement linéaire, et soit au moins un polymère comportant au moins un monomère à insaturation éthylénique et à groupement sulfonique, soit au moins une poudre organique, soit un mélange des deux., et à l'utilisation de la dite composition dans les domaines cosmétique et dermatologique, en particulier pour le soin, le traitement, le nettoyage et/ou le maquillage de la peau du corps ou du visage, des cheveux et/ou des lèvres.

Pour apporter à la fois un effet hydratant et un effet émollient, les compositions cosmétiques actuelles se présentent le plus souvent sous la forme d'une émulsion comportant une phase aqueuse et une phase huileuse. Selon le sens de la dispersion, il peut s'agir d'une émulsion du type huile-dans-eau (H/E) constituée d'une phase continue dispersante aqueuse et d'une phase discontinue dispersée huileuse, ou d'une émulsion du type eau-dans-huile (E/H) constituée d'une phase continue dispersante huileuse et d'une phase discontinue dispersée aqueuse. Les émulsions H/E sont les plus demandées dans le domaine cosmétique du fait qu'elles comportent, comme une phase externe, une phase aqueuse, ce qui leur confère, lors de l'application sur la peau, un toucher plus frais, moins gras et plus léger que les émulsions E/H.

Toutefois, le toucher des compositions cosmétiques et notamment de ces émulsions peut laisser à désirer car il manque souvent de douceur à l'application. Pour remédier à cet inconvénient, on peut ajouter des agents de texture qui confèrent de la douceur à la composition les contenant. Ainsi, le document EP-A-874017 décrit des particules de copolymère siliconé bloc sous forme de dispersions silicone-dans-eau, appropriées pour être incorporées dans des produits de soin et y apporter de la douceur.

Toutefois, si l'incorporation d'une telle dispersion apporte bien de la douceur, elle apporte aussi un effet collant lors de l'application sur la peau, désagréable pour l'utilisateur.

L'objectif de l'invention est de pouvoir réaliser des émulsions présentant de bonnes propriétés cosmétiques sans avoir les inconvénients de l'art antérieur.

La demanderesse a découvert de façon inattendue que l'utilisation d'un polymère comportant au moins un monomère à insaturation éthylénique et à groupement sulfonique et notamment d'un polymère ou copolymère d'acide 2-acrylamido-2-méthylpropane sulfonique (AMPS), ou bien l'utilisation d'une poudre organique dans une composition contenant des particules en émulsion, telles que décrites dans le document EP-A-874017, permettait d'enlever l'effet collant apporté par cette émulsion tout en gardant l'effet de douceur, et permettait en particulier de réaliser des compositions cosmétiques et notamment des émulsions notamment huile-dans-eau ayant de très bonnes propriétés cosmétiques.

Le document US-A-2001/0041768 décrit des émulsions E/H dont la phase huileuse contient des particules d'élastomère d'organopolysiloxane réticulé et dont la phase aqueuse contient un polymère d'AMPS.

Le document EP-A-1,013,700 décrit des émulsions H/E contenant un copolymère de silicone non réticulé linéaire.

La présente invention a ainsi pour objet une composition comprenant dans un milieu physiologiquement acceptable, au moins une phase aqueuse comprenant en dispersion, des particules de copolymère siliconé bloc substantiellement linéaire, et soit au moins un polymère comportant au moins un monomère à insaturation éthylénique et à groupement sulfonique, soit au moins une poudre organique, soit un mélange d'au moins un polymère comportant au moins un monomère à insaturation éthylénique et à groupement sulfonique et d'au moins une poudre organique.

La composition de l'invention étant destinée notamment à une application topique comprend un milieu physiologiquement acceptable, c'est-à-dire un milieu compatible avec toutes les matières kératiniques telles que la peau, les ongles, les muqueuses et les cheveux ou toute autre zone cutanée du corps. Elle peut constituer notamment une composition cosmétique ou dermatologique.

Dans la présente demande, on entend par "copolymère substantiellement linéaire", un copolymère comportant peu de ramifications, et généralement moins de 2 % en mole des unités siloxane.

Par ailleurs, on entend par "particules" les globules de copolymère siliconé bloc qui sont en dispersion dans de l'eau et forme une émulsion silicone-dans-eau.

La composition obtenue selon l'invention présente l'avantage d'avoir une texture homogène, très douce, non collante et fraîche à l'application, donc très agréable à utiliser.

En outre, il a été observé que les particules de copolymère siliconé bloc substantiellement linéaire en dispersion aqueuse améliore la dispersion des poudres organiques dans la composition. Aussi, la présente invention a aussi pour objet l'utilisation d'une dispersion aqueuse de particules de copolymère siliconé bloc substantiellement linéaire, pour améliorer la dispersion de poudres organiques dans une composition comprenant au moins une phase aqueuse.

En outre, la dispersion de particules de copolymère siliconé substantiellement linéaire utilisée dans la composition selon l'invention permet de préparer des compositions et notamment des émulsions huile-dans-eau, qui restent stables dans le temps à température ambiante ou à des températures plus élevées, et de conserver ces propriétés, quelle que soit la fluidité de la composition. On peut donc ainsi préparer aussi bien des émulsions épaisses particulièrement efficaces pour le traitement des peaux sèches que des émulsions très fluides. La viscosité des émulsions peut donc varier dans une large mesure et aller par exemple de 0,05 Pa.s à 20 Pa.s, de préférence de 0,05 à 10 Pa.s, ces viscosités étant mesurées à environ 25°C à l'aide du viscosimètre "Rhéomat 180" qui est, de manière générale, équipé d'un mobile 2 pour les gammes de viscosités de 0,02 Pa.s à 0,7 Pa.s, d'un mobile 3 pour les gammes de viscosités de 0,2 Pa.s à 4 Pa.s, et d'un mobile 4 pour les gammes de viscosités de 2 Pa.s à 23 Pa.s.

Par ailleurs, la composition selon l'invention permet d'obtenir des compositions stables, même quand elles contiennent des actifs hydrophiles sensibles à l'oxydation qui ont tendance non seulement à se déstabiliser mais aussi à déstabiliser les compositions les contenant, et notamment les émulsions. Ainsi, la dispersion de particules de copolymère bloc présente l'avantage de stabiliser les actifs hydrophiles sensibles à l'oxydation et de stabiliser les compositions contenant de tels actifs.

### Copolymère siliconé bloc

Le copolymère siliconé constituant les globules ou particules en dispersion dans la phase aqueuse est un copolymère bloc substantiellement linéaire, c'est-à-dire un copolymère non réticulé, obtenu par extension de chaîne et non par réticulation.

La dispersion aqueuse de particules de copolymère bloc est une émulsion silicone dans eau (Sil/E), dont les globules huileux sont constitués d'un silicone de viscosité élevée, de sorte que ces globules semblent former comme "des particules souples".

La composition peut comprendre des dispersions d'un ou plusieurs copolymères siliconés blocs substantiellement linéaires. Ces copolymères siliconés blocs sont présents dans la composition de l'invention dans des concentrations en matière active pouvant varier largement selon les autres ingrédients de la composition et le but recherché. La concentration en matière active (M.A.) de copolymère siliconé bloc va de préférence de 0,01 à 15 % en poids, mieux de 0,1 à 10 % et encore mieux de 0,5 à 5 % en poids par rapport au poids total de la composition.

La taille des particules de copolymère siliconé bloc peut varier largement. De manière préférée dans la présente demande, les particules de copolymère siliconé présentent généralement une taille moyenne en nombre inférieure ou égale à 2 microns, et de préférence inférieure ou égale à 1 micron.

Les dispersions aqueuses de particules de copolymères siliconés blocs substantiellement linéaires, utilisées selon l'invention peuvent être choisies notamment parmi celles décrites dans le document EP-A-874017, dont l'enseignement est incorporé ici par référence. Selon ce document, on peut notamment obtenir les copolymères siliconés constituant ces particules par réaction d'extension de chaîne, en présence d'un catalyseur, à partir d'au moins :
- (a) un polysiloxane (i) ayant au moins un groupe réactif et de préférence un ou deux groupes réactifs par molécule ; et
- (b) un composé organosiliconé (ii) qui réagit avec le polysiloxane (i) par réaction d'extension de chaîne.

En particulier, le polysiloxane (i) est choisi parmi les composés de formule (I) : 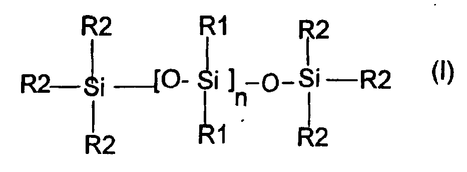 dans laquelle R₁ et R₂ indépendamment les uns des autres représentent un groupe hydrocarboné ayant de 1 à 20 atomes de carbone et de préférence de 1 à 10 atomes de carbone, tel que méthyle, éthyle, propyle ou butyle, ou un groupe aryle tel que phényle, ou un groupe réactif, n est un nombre entier supérieur à 1, à la condition qu'il y ait en moyenne entre un et deux groupes réactifs par polymère.

On entend par « groupe réactif » tout groupe susceptible de réagir avec le composé organosiliconé (ii) pour former un copolymère bloc. Comme groupes réactifs, on peut citer l'hydrogène ; les groupes aliphatiquement insaturés et notamment vinyle, allyle ou hexanyle ; le groupe hydroxyle ; les groupes alcoxy tels que méthoxy, éthoxy ou propoxy ; les groupes alcoxy-alcoxy ; le groupe acétoxy ; les groupes aminés, et leurs mélanges. De préférence, plus de 90 % et mieux plus de 98% de groupes réactifs sont en bout de chaîne, c'est-à-dire que les radicaux R₂ constituent généralement plus de 90 % et même 98% des groupes réactifs.

De préférence, n est tel que les polysiloxanes ont une viscosité allant d'environ 1 à 1.10⁶ mm²/sec à 25°C. n peut être notamment un nombre entier allant de 5 à 30, de préférence de 10 à 30 et mieux de 15 à 25.

Les polysiloxanes de formule (I) sont des polymères substantiellement linéaires, c'est-à-dire comportant peu de ramifications, et généralement moins de 2 % en mole des unités siloxane. Par ailleurs, les groupes R₁ et R₂ peuvent être éventuellement substitués par des groupes aminés, des groupes époxy, des groupes comportant du soufre, du silicium ou de l'oxygène.

De préférence, au moins 80 % des groupes R₁ sont des groupes alkyle et mieux des groupes méthyle.

De préférence, le groupe réactif R₂ en bout de chaîne est un groupe aliphatiquement insaturé et notamment vinyle.

Comme polysiloxanes (i), on peut citer notamment le diméthylvinyl-siloxy-polydiméthylsiloxane, composé de formule (I) dans laquelle les radicaux R₁ sont des radicaux méthyle, et, en bout de chaîne, le radical R₂ est un radical vinyle tandis que les deux autres radicaux R₂ sont des radicaux méthyle.

Le composé organosiliconé (ii) peut être choisi parmi les polysiloxanes de formule (I) ou les composés agissant comme agent d'extension de chaîne. Si c'est un composé de formule (I), le polysiloxane (i) comportera un premier groupe réactif et le composé organosiliconé (ii) comportera un second groupe réactif qui réagira avec le premier. Si c'est un agent d'extension de chaîne, ce peut être un silane, un siloxane (disiloxane ou trisiloxane) ou un silazane. De préférence, le composé organosiliconé (ii) est un organohydrogenopolysiloxane liquide de formule (II) : 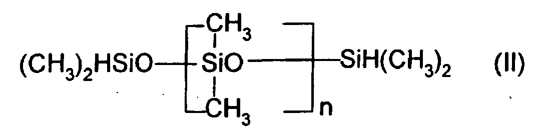 où n est un nombre entier supérieur à 1 et de préférence supérieur à 10, et par exemple allant de 5 à 30, de préférence de 10 à 30 et mieux de 15 à 25. Selon un mode particulier de réalisation de l'invention, n est égal à 20.

Les copolymères blocs siliconés utilisés selon l'invention sont avantageusement exempts de groupe oxyalkyléné, notamment exempts de groupes oxyéthylénés et/ou oxypropylénés.

Le catalyseur de la réaction entre le polysiloxane et le composé organosiliconé peut être choisi parmi les métaux et notamment parmi le platine, le rhodium, l'étain, le titane, le cuivre et le plomb. Il s'agit de préférence du platine ou du rhodium.

La dispersion de particules de copolymère siliconé utilisée selon l'invention peut être notamment obtenue par exemple par mélange de (a) l'eau, (b) au moins un émulsifiant, (c) le polysiloxane (i), (d) le composé organosiliconé (ii) et (e) un catalyseur. De préférence, l'un des constituants (c), (d) ou (e) est ajouté en dernier dans le mélange, afin que la réaction d'extension de chaîne ne commence que dans la dispersion.

Comme émulsifiants susceptibles d'être utilisés dans le procédé de préparation décrit ci-dessus pour obtenir la dispersion aqueuse de particules, on peut citer les émulsifiants non ioniques ou ioniques (anioniques, cationiques ou amphotères). II s'agit de préférence d'émulsifiants non ioniques qui peuvent être choisis parmi les polyalkylène glycols éthers d'alcool gras, comportant de 8 à 30 atomes de carbone et de préférence de 10 à 22 atomes de carbone ; les alkylesters de sorbitan polyoxyalkylénés et notamment polyoxyéthylénés, où le radical alkyle comporte de 8 à 30 atomes de carbone et de préférence de 10 à 22 atomes de carbone ; les alkylesters polyoxyalkylénés et notamment polyoxyéthylénés, où le radical alkyle comporte de 8 à 30 atomes de carbone et de préférence de 10 à 22 atomes de carbone ; les polyéthylène glycols ; les polypropylène glycols ; les diéthylène glycols ; et leurs mélanges. La quantité d'émulsifiant(s) est généralement de 1 à 30 % en poids par rapport au poids total du mélange de réaction.

L'émulsifiant utilisé pour obtenir la dispersion aqueuse de particules est de préférence choisi parmi les polyéthylène glycol éthers d'alcools gras et leurs mélanges, et notamment les polyéthylène glycol éthers d'alcools comportant 12 ou 13 atomes de carbone et de 2 à 100 motifs oxyéthylénés et de préférence de 3 à 50 motifs oxyéthylénés, et leurs mélanges. On peut citer par exemple le C₁₂₋C₁₃ Pareth-3, le C₁₂-C₁₃ Pareth-23 et leurs mélanges.

Selon un mode particulier de réalisation de l'invention, la dispersion de particules de copolymère siliconé est obtenue à partir de diméthylvinyl-siloxy-polydiméthylsiloxane (ou divinyldimethicone) comme composé (i), et du composé de formule (II) avec de préférence n=20, comme composé (ii), de préférence en présence d'un catalyseur de type platine, et la dispersion de particules est de préférence obtenue en présence de C₁₂-C₁₃ Pareth-3 et C₁₂-C₁₃ Pareth-23 comme émulsifiants.

Comme dispersion de particules de copolymère siliconé, on peut utiliser notamment le produit commercialisé sous la dénomination HMW 2220 par la société Dow Corning (nom CTFA : divinyldimethicone/dimethicone Copolymer / C₁₂-C₁₃ Pareth-3 / C₁₂-C₁₃ Pareth-23), qui est une dispersion aqueuse à 60 % de copolymère divinyldimethicone / dimethicone, contenant du C₁₂-C₁₃ Pareth-3 et du C₁₂-C₁₃ Pareth-23, la dite dispersion comprenant environ 60 % en poids de copolymère ; 2,8 % en poids de C₁₂-C₁₃ Pareth-23 ; 2 % en poids de C₁₂-C₁₃ Pareth-3 ; 031 % en poids de conservateurs, le reste à 100 % étant de l'eau.

### Polymères comportant au moins un monomère à insaturation éthylénique et à groupement sulfonique

Les polymères utilisés conformément à l'invention sont des homopolymères ou copolymères comportant au moins un monomère à insaturation éthylénique et à groupement sulfonique, pouvant être sous forme libre ou partiellement ou totalement neutralisée. Ces polymères peuvent éventuellement comprendre au moins une partie hydrophobe et constituer alors un polymère amphiphile.

De façon préférentielle, les polymères conformes à l'invention peuvent être neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés » des polymères totalement ou pratiquement totalement neutralisés, c'est-à-dire neutralisés à au moins 90 %.

Les polymères utilisés dans la composition de l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Ces polymères selon l'invention peuvent être réticulés ou non-réticulés. De préférence, on choisit des polymères réticulés.

Les monomères à insaturation éthylénique et à groupement sulfonique du polymère utilisé dans la composition de l'invention sont choisis notamment parmi l'acide vinylsulfonique, l'acide styrènesulfonique, les acides (méth)acrylamido-(C₁-C₂₂)alkylsulfoniques, les acides N-(C₁-C₂₂)alkyl-(méth)acrylamido-(C₁-C₂₂)alkylsulfoniques comme l'acide undécyl-acrylamido-méthane-sulfonique, ainsi que leurs formes partiellement ou totalement neutralisées, et leurs mélanges.

Selon un mode préféré de réalisation de l'invention, on utilise comme monomères à insaturation éthylénique et à groupement sulfonique, les acides (méth)acrylamido(C₁-C₂₂)alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide 2-méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique, ainsi que leurs formes partiellement ou totalement neutralisées, et leurs mélanges.

Plus particulièrement, on utilise l'acide 2-acrylamido-2-méthylpropane-sulfonique (AMPS) ainsi que ses formes partiellement ou totalement neutralisées.

Lorsque les polymères sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

Lorsque les polymères utilisés sont des homopolymères, ils ne comportent que des monomères à insaturation éthylènique et à groupement sulfonique et, s'ils sont réticulés, un ou plusieurs agents de réticulation.

Ces homopolymères sont généralement réticulés et neutralisés, et ils peuvent être obtenus selon le procédé de préparation comprenant les étapes suivantes :
(a) on disperse ou on dissout le monomère tel que l'acide 2-acrylamido 2-méthylpropane sulfonique sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque NH₃, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Comme polymères de ce type, on peut citer notamment l'homopolymère réticulé et neutralisé d'acide 2-acrylamido 2-méthylpropane sulfonique, commercialisé par la société Hoechst sous la dénomination commerciale « Hostacerin AMPS » (nom CTFA : ammonium polyacryldimethyltauramide).

Le polymère peut être aussi un homopolymère amphiphile choisi parmi les polymères amphiphiles statistiques d'AMPS modifiés par réaction avec une n-monoalkylamine ou une di-n-alkylamine en C₆-C₂₂, tels que ceux décrits dans la demande de brevet WO-A-00/31154, qui sont des homopolymères greffés.

Lorsque les polymères utilisés sont des copolymères, ils sont obtenus à partir de des monomères à insaturation éthylénique et à groupement sulfonique et d'autres monomères à insaturation éthylénique, c'est-à-dire de monomères à insaturation éthylénique sans groupement sulfonique.

Les monomères à insaturation éthylénique et à groupement sulfonique sont choisis parmi ceux décrits ci-dessus.

Les monomères à insaturation éthylénique sans groupement sulfonique peuvent être choisis parmi les monomères hydrophiles, les monomères hydrophobes et leurs mélanges. Quand le polymère contient des monomères hydrophobes, il constitue un polymère amphiphile.

Les monomères hydrophiles à insaturation éthylénique peuvent être choisis par exemple parmi les acides (méth)acryliques, leurs dérivés alkyl substitués en β ou leurs esters obtenus avec des monoalcools ou des mono- ou poly-alkylèneglycols, les (méth)acrylamides, la vinylpyrrolidone, le vinylformamide, l'anhydride maléique, l'acide itaconique ou l'acide maléique ou les mélanges de ces composés.

Comme copolymères obtenus à partir de monomères à insaturation éthylénique et à groupement sulfonique et de monomères hydrophiles à insaturation éthylénique, on peut citer par exemple ceux obtenus à partir d'AMPS et d'acrylamide ou de méthylacrylamide, comme par exemple le copolymère acrylamide/acrylamido-2-methyl propane sulfonate de sodium en émulsion inverse à 40% dans le polysorbate, commercialisé sous la dénomination SIMULGEL 600 par la société SEPPIC. On peut aussi citer les copolymères d'AMPS et de vinylpyrrolidone ou de vinylformamide, tels que les produits commercialisés sous les dénominations ARISTOFLEX AVC par la société CLARIANT.

Quand les monomères à insaturation éthylénique et à groupement sulfonique sont copolymérisés avec des monomères hydrophobes à insaturation éthylénique comportant une chaîne grasse, le polymère obtenu est amphiphile, c'est-à-dire qu'il comporte à la fois une partie hydrophile et une partie hydrophobe.

Ces polymères amphiphiles peuvent contenir en outre un ou plusieurs monomères éthyléniquement insaturés ne comportant pas de chaîne grasse, tels que les acides (méth)acryliques, leurs dérivés alkyl substitués en β ou leurs esters obtenus avec des monoalcools ou des mono- ou poly-alkylèneglycols, les (méth)acrylamides, la vinylpyrrolidone, le vinylformamide, l'anhydride maléique, l'acide itaconique ou l'acide maléique ou les mélanges de ces composés.

Comme polymères amphiphiles, on peut utiliser notamment ceux obtenus à partir d'AMPS et d'au moins un monomère hydrophobe à insaturation éthylénique comportant au moins une partie hydrophobe ayant de 6 à 50 atomes de carbone, plus préférentiellement de 6 à 22 atomes de carbone, encore plus préférentiellement de 6 à 18 atomes de carbone et plus particulièrement 12 à 18 atomes de carbone.

Ces copolymères sont décrits notamment dans la demande de brevet EP-A-750899, le brevet US-A-5089578 et dans les publications de Yotaro Morishima suivantes :
- « Self-assembling amphiphilic polyelectrolytes and their nanostructures - Chinese Journal of Polymer Science Vol. 18, N°40, (2000), 323-336 »;
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte: salt effects on rheological behavior - Langmuir, 2000, Vol. 16, N°12, 5324-5332 » ;
   « Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

Les monomères hydrophobes à insaturation éthylénique de ces copolymères particuliers sont choisis de préférence parmi les acrylates, alkylacrylates, acrylamides ou alkylacrylamides de formule (III) suivante : 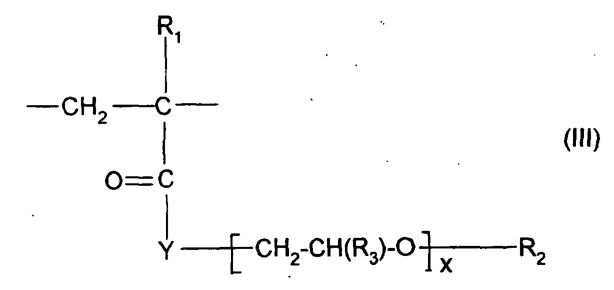 dans laquelle R₁ et R₃, identiques ou différents, désignent un atome d'hydrogène ou un radical alkyle substantiellement linéaire ou ramifié en C₁-C₆ (de préférence méthyle) ; Y désigne O ou NH ; R₂ désigne un radical hydrocarboné hydrophobe comportant au moins de 6 à 50 atomes de carbone, plus préférentiellement de 6 à 22 atomes de carbone, encore plus préférentiellement de 6 à 18 atomes de carbone et plus particulièrement de 12 à 18 atomes de carbone ; x désigne un nombre de moles d'oxyde d'alkylène et varie de 0 à 100.

Le radical R₂ est choisi de préférence parmi les radicaux alkyles en C₆-C₁₈ substantiellement linéaires (par exemple n-hexyle, n-octyle, n-décyle, n-hexadécyle, n-dodécyle, n-octadécyle) ramifiés ou cycliques (par exemple cyclododécane (C₁₂) ou adamantane (C₁₀)) ; les radicaux alkylperfluorés en C₆₋C₁₈ (par exemple le groupement de formule -(CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles substantiellement linéaires et plus particulièrement le radical n-dodécyle, n-hexadecyle ou n-octadécyle, et leurs mélanges.

Selon une forme particulièrement préférée de l'invention, le monomère de formule (III) comporte au moins un motif oxyde d'alkylène (x ≥ 1) et de préférence plusieurs motifs oxyde d'alkylène (x > 1) constituant une chaîne polyoxyalkylénée: La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés (ou nombre de moles d'oxyde d'alkylène) varie en général de 3 à 100, plus préférentiellement de 3 à 50 et encore plus préférentiellement de 7 à 25.

Parmi ces polymères, on peut citer :
- les copolymères réticulés ou non réticulés, neutralisés ou non, comportant de 15 à 60 % en poids de motifs AMPS et de 40 à 85 % en poids de motifs (C₈-C₁₆)alkyl(méth)acrylamide ou de motifs (C₈-C₁₆)alkyl(méth)acrylate par rapport au polymère, tels que ceux décrits dans la demande EP-A-750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, par rapport au polymère, tels que ceux décrits dans le brevet US-A-5,089,578.

Comme polymères amphiphiles, on peut également citer les copolymères d'AMPS totalement neutralisé et de méthacrylate de n-dodécyle, de n-hexadécyle et/ou de n-octadécyle, ainsi que les copolymères d'AMPS et de n-dodécylméthacrylamide, non-réticulés et réticulés, tels que ceux décrits dans les articles de Morishima cités ci-dessus.

On peut citer plus particulièrement les copolymères constitués de motifs acide 2-acrylamido-2-méthylpropane-sulfonique (AMPS) de formule (IV) suivante : 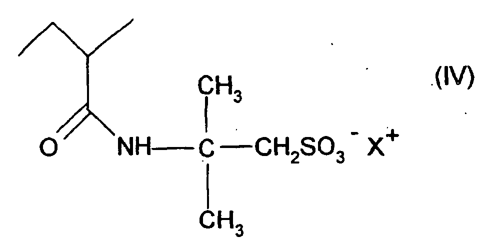 dans laquelle X⁺ est un proton, un cation de métal alcalin, un cation alcalino-terreux ou l'ion ammonium,
et de motifs de formule (V) suivante : 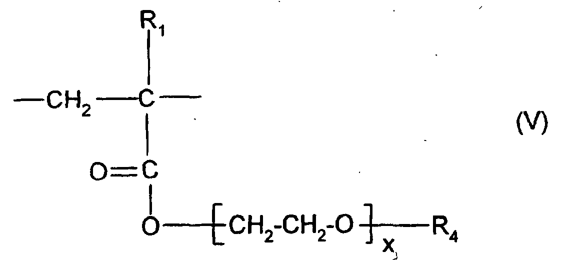 dans laquelle x désigne un nombre entier variant de 3 à 100, de préférence de 3 à 50 et plus préférentiellement de 7 à 25 ; R₁ a la même signification que celle indiquée ci-dessus dans la formule (I) et R₄ désigne un alkyle substantiellement linéaire ou ramifié en C₆-C₂₂ et plus préférentiellement en C₁₀-C₂₂.

Les polymères amphiphiles de ce type particulièrement préférés sont ceux pour lesquels x = 25, R₁ désigne méthyle et R₄ représente n-dodécyle ; ils sont décrits dans les articles de Morishima mentionnés ci-dessus. D'autres polymères préférés sont ceux pour lesquels x = 8 ou 25, R₁ désigne méthyle et R₄ représente n-hexadécyle (C₁₆), n-octadécyle (C₁₈), ou n-dodécyle (C₁₂) ou leurs mélanges.

Les polymères pour lesquels X⁺ désigne le sodium ou l'ammonium sont plus particulièrement préférés.

Les polymères amphiphiles préférés utilisés dans la composition conforme à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le chlorhydrate de 2,2-azobis-[2-amidinopropane] (ABAH = 2,2-Azo-Bis-[2-Amidinopropane] Hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ces polymères amphiphiles peuvent être notamment obtenus par polymérisation radicalaire en milieu tert-butanol dans lequel ils précipitent. En utilisant la polymérisation par précipitation dans le tert-butanol, il est possible d'obtenir une distribution de la taille des particules du polymère particulièrement favorable pour ses utilisations.

La réaction peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.

Selon ce procédé, on a notamment polymérisé l'acide 2-acrylamido-2-méthylpropane-sulfonique (AMPS) ou l'un de ses sels de sodium ou d'ammonium, avec un ester de l'acide (méth)acrylique et
- d'un alcool en C₁₀-C₁₈ oxyéthyléné par 8 moles d'oxyde d'éthylène (GENAPOL® C-080 de la société CLARIANT),
- d'un alcool oxo en C₁₁ oxyéthyléné par 8 moles d'oxyde d'éthylène (GENAPOL® UD-080 de la société CLARIANT),
- d'un alcool oxo en C₁₁ oxyéthyléné par 7 moles d'oxyde d'éthylène (GENAPOL® UD-070 de la société CLARIANT),
- d'un alcool en C₁₂-C₁₄ oxyéthyléné par 7 moles d'oxyde d'éthylène (GENAPOL® LA-070 de la société CLARIANT),
- d'un alcool en C₁₂-C₁₄ oxyéthyléné par 9 moles d'oxyde d'éthylène (GENAPOL® LA-090 de la société CLARIANT),
- d'un alcool en C₁₂-C₁₄ oxyéthyléné par 11 moles d'oxyde d'éthylène (GENAPOL® LA-110 de la société CLARIANT),
- d'un alcool en C₁₆-C₁₈ oxyéthyléné par 8 moles d'oxyde d'éthylène (GENAPOL® T-080 de la société CLARIANT),
- d'un alcool en C₁₆-C₁₈ oxyéthyléné par 15 moles d'oxyde d'éthylène (GENAPOL® T-150 de la société CLARIANT),
- d'un alcool en C₁₆-C₁₈ oxyéthyléné par 11 moles d'oxyde d'éthylène (GENAPOL® T-110 de la société CLARIANT),
- d'un alcool en C₁₆-C₁₈ oxyéthyléné par 20 moles d'oxyde d'éthylène (GENAPOL® T-200 de la société CLARIANT),
- d'un alcool en C₁₈-C₁₈ oxyéthyléné par 25 moles d'oxyde d'éthylène (GENAPOL® T-250 de la société CLARIANT),
- d'un alcool en C₁₈-C₂₂ oxyéthyléné par 25 moles d'oxyde d'éthylène et/ou d'un alcool iso-C₁₆-C₁₈ oxyéthyléné par 25 moles d'oxyde d'éthylène.

La concentration molaire en % des motifs de formule (II) et des motifs de formule (III) dans les polymères selon l'invention varie en fonction de l'application cosmétique souhaitée et des propriétés rhéologiques de la formulation recherchées: Elle peut varier entre 0,1 et 99,9 % en moles.

De préférence pour les polymères les plus hydrophobes, la proportion molaire en motifs de formule (I) ou (III) varie de 50,1 à 99,9 %, plus particulièrement de 70 à 95 % et encore plus particulièrement de 80 à 90 %.

De préférence pour les polymères peu hydrophobes, la proportion molaire en motifs de formule (I) ou (III) varie de 0,1 à 50 %, plus particulièrement de 5 à 25 % et encore plus particulièrement de 10 à 20 %.

La distribution des monomères dans les polymères de l'invention peut être, par exemple, alternée, bloc (y compris multibloc) ou quelconque.

Les polymères comportant au moins un monomère à insaturation éthylénique et à groupement sulfonique, utilisés dans la composition conforme à l'invention sont généralement présents dans des quantités en matière active allant de 0,01 à 20 % en poids, plus préférentiellement de 0,1 à 10 % en poids, encore plus préférentiellement de 0,1 à 5 % en poids et plus particulièrement encore de 0,5 à 2 % en poids de par rapport au poids total de la composition.

### Poudre organique

Comme poudres organiques (appelées aussi charges organiques) pouvant être utilisées dans la composition de l'invention, on peut citer par exemple les particules de polyamide et notamment la poudre de NYLON 12, comme les produits commercialisés sous les dénominations ORGASOL par la société Atochem ; les poudres et billes de polyéthylène telles que celles commercialisées sous les dénominations ACUMIST (Acumist B-6, Acumist B-12) par la société Allied et celles commercialisées sous les dénominations MICROTHENE par la société Equistar ; les microsphères à base de copolymères acryliques ou méthacryliques, telles que celles en copolymère diméthacrylate d'éthylène glycol/methacrylate de lauryle, vendues par la société Dow Corning sous la dénomination de POLYTRAP, ou celles en copolymère méthacrylate de méthyle / diméthacrylate d'éthylène glycol, commercialisées sous la dénomination Microsphères M305 par la société Matsumoto ; les microsphères de polyméthacrylate de méthyle commercialisées sous la dénomination MICROSPHERE M-100 et M305 par la société Matsumoto ou sous les dénominations COVABEAD par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous les dénominations TOSPEARL par la société Toshiba Silicone ; et leurs mélanges.

Selon les cas, la poudre organique peut être introduite dans la composition après mélange des autres constituants et, par exemple, dans le cas d'une émulsion après préparation de l'émulsion, ou bien, si une phase huileuse est présente, dans la phase huileuse de la composition. Elle peut aussi être introduite pendant la préparation de l'émulsion, dans la phase aqueuse ou dans la phase huileuse.

Les poudres organiques utilisées dans la composition conforme à l'invention sont généralement présentes dans des quantités en matière active allant de 0,01 à 30 % en poids, plus préférentiellement de 0,1 à 20 % en poids, encore plus préférentiellement de 1 à 10 % en poids par rapport au poids total de la composition.

Les compositions selon l'invention peuvent se présenter sous toutes les formes galéniques classiquement utilisées pour une application topique et notamment sous forme de gels aqueux, de solutions aqueuses ou hydroalcooliques. Elles peuvent aussi par ajout d'une phase grasse ou huileuse se présenter sous forme de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

Selon un mode préféré de réalisation de l'invention, la composition se présente sous forme d'une émulsion et notamment d'une émulsion H/E.

En outre, les compositions utilisées selon l'invention peuvent être plus ou moins fluides et avoir l'aspect d'un gel, d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de poudre, sous forme coulée, en coupelle ou sous forme de stick.

La phase aqueuse des compositions selon l'invention comprend au moins de l'eau. Elle peut n'être constituée que de la dispersion aqueuse des particules de copolymères siliconés blocs ou comprendre d'autres constituants hydrosolubles ou hydrodispersibles. Dans les compositions anhydres, c'est-à-dire comportant moins de 5 % en poids d'eau par rapport au poids totale de la composition, la phase aqueuse n'est constituée que de la dispersion aqueuse des particules de copolymères siliconés blocs. Selon la forme galénique de la composition, la quantité de phase aqueuse peut aller de 0,1 à 99 % en poids, de préférence de 0,5 à 98 % en poids, mieux de 30 à 95 % en poids, et encore mieux de 40 à 95 % en poids par rapport au poids total de la composition. Cette quantité dépend de la forme galénique de la composition désirée. La quantité d'eau peut représenter tout ou une partie de la phase aqueuse, et elle est généralement d'au moins 30 % en poids par rapport au poids total de la composition, notamment quand la composition se présente sous forme d'émulsion.

Les compositions de l'invention peuvent contenir dans la phase aqueuse ou dans la phase huileuse si elle comporte une phase huileuse, un ou plusieurs solvants organiques, hydrophiles, lipophiles et/ou amphiphiles, physiologiquement acceptables, c'est-à-dire bien tolérés et donnant un toucher cosmétiquement acceptable.

Les solvants organiques peuvent représenter de 0,5 à 50 % et de préférence de 2 à 20 % du poids total de la composition. Les solvants organiques peuvent être choisis dans le groupe constitué par les solvants organiques hydrophiles, les solvants organiques lipophiles, les solvants amphiphiles, ou leurs mélanges.

Parmi les solvants organiques, on peut citer par exemple les mono-alcools inférieurs substantiellement linéaires ou ramifiés ayant de 1 à 8 atomes de carbone, comme l'éthanol, le propanol, le butanol, l'isopropanol, l'isobutanol ; les polyols tels que le propylène glycol, l'isoprène glycol, le butylène glycol, le propylène glycol; le glycérol, le sorbitol ; les mono- ou di-alkyle isosorbide dont les groupements alkyle ont de 1 à 5 atomes de carbone comme le diméthyl isosorbide ; les polyéthylène glycols notamment ceux ayant de 6 à 80 oxydes d'éthylène, tels que le polyéthylène glycol 32 OE ; les éthers d'éthylène glycol comme le diéthylène glycol mono-méthyl ou mono-éthyl éther; les éthers de propylène glycol comme le dipropylène glycol méthyl éther ; les esters et éthers de polyol, tels que les esters de polypropylène glycol (PPG) et plus spécialement les esters de polypropylène glycol (PPG) et d'acide gras, les éthers de PPG et d'alcool gras comme le PPG-23 oléyl éther et le PPG-36 oléate ; les esters d'acide gras et d'alkyle, tels que l'adipate de diisopropyle, l'adipate de dioctyle, les benzoates d'alkyle ; et leurs mélanges.

Lorsque la composition est sous forme d'émulsion, la proportion de la phase huileuse de l'émulsion peut aller par exemple de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90^{R} par la société Goldschmidt, ou le mélange polyglycéryl-4 isostéarate/cétyl diméthicone copolyol/hexyllaurate vendu sous la dénomination ABIL WE 09 par la société Goldschmidt. On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan Gl 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Pour les émulsions H/E, on peut citer par exemple comme émulsionnants, les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers d'alcools gras ; les éthers de sucre et d'alcools gras en C₈-C₂₄, et leurs mélanges.

Comme ester de glycéryle et d'acide gras, on peut citer notamment le stéarate de glycéryle (mono-, di- et/ou tri-stéarate de glycéryle) (nom CTFA : glyceryl stearate) ou le ricinoléate de glycéryle, et leurs mélanges.

Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom CTFA : PEG-50 stéarate), le monostéarate de polyéthylène glycol 100 OE (nom CTFA : PEG-100 stéarate et leurs mélanges.

On peut aussi utiliser des mélanges de ces tensioactifs, comme par exemple le produit contenant du Glyceryl stéarate et du PEG-100 stearate, commercialisé sous la dénomination ARLACEL 165 par la société Uniqema, et le produit contenant du Glyceryl stéarate (mono-distéarate de glycéryle) et du stéarate de potassium, commercialisé sous la dénomination TEGIN par la société Goldschmidt (nom CTFA : glyceryl stéarate SE).

Comme ester d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmitate de méthylglucose ou d'éthylglucose, les esters gras de méthylglucoside et plus spécialement le diester de méthylglucoside et d'acide oléique (nom CTFA : Methyl glucose dioleate) ; l'ester mixte de méthylglucoside et du mélange acide oléique / acide hydroxystéarique (nom CTFA : Methyl glucose dioleate/hydroxystearate) ; l'ester de méthylglucoside et d'acide isostéarique (nom CTFA : Methyl glucose isostearate) ; l'ester de méthylglucoside et d'acide laurique (nom CTFA : Methyl glucose laurate) ; le mélange de monoester et de diester de méthylglucoside et d'acide isostéarique (nom CTFA : Methyl glucose sesqui-isostearate) ; le mélange de monoester et de diester de méthylglucoside et d'acide stéarique (nom CTFA : Methyl glucose sesquistearate) et en particulier le produit commercialisé sous la dénomination Glucate SS par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom CTFA : PEG-20 methyl glucose distearate) tel que le produit commercialisé sous la dénomination Glucam E-20 distearate par la société AMERCHOL ; l'éther de polyéthylène glycol du mélange de monoester et de diester de méthyl glucose et d'acide stéarique à environ 20 moles d'oxyde d'éthylène (nom CTFA : PEG-20 methyl glucose sesquistearate) et en particulier le produit commercialisé sous la dénomination Glucamate SSE-20 par la société AMERCHOL et celui commercialisé sous la dénomination Grillocose PSE-20 par la société GOLDSCHMIDT, et leurs mélanges.

Comme esters de sucrose, on peut citer par exemple le palmito-stéarate de saccharose, le stéarate de saccharose et le mono laurate de saccharose.

Comme éthers d'alcools gras, on peut citer par exemple les éthers de polyéthylène glycol et d'alcool gras comportant de 8 à 30 atomes de carbone, et notamment de 10 à22 atomes de carbone, tels que les éthers de polyéthylène glycol et d'alcools cétylique, stéarylique, cetéarylique (mélange d'alcools cétylique et stéarylique). On peut citer par exemple les éthers comportant de 1 à 200 et de préférence de 2 à 100 groupes oxyéthylénés, tels que ceux de nom CTFA Ceteareth-20, Ceteareth-30, et leurs mélanges.

Comme éthers de sucre, on peut citer notamment les alkylpolyglucosides, et par exemple le decylglucoside comme le produit commercialisé sous la dénomination MYDOL 10 par la société Kao Chemicals, le produit commercialisé sous la dénomination PLANTAREN 2000 par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; le coco-glucoside comme le produit commercialisé sous la dénomination PLANTACARE 818/UP par la société Henkel ; le cétostéaryl glucoside éventuellement en mélange avec l'alcool cétostéarylique, commercialisé par exemple sous la dénomination MONTANOV 68 par la société Seppic, sous la dénomination TEGO-CARE CG90 par la société Goldschmidt et sous la dénomination EMULGADE KE3302 par la société Henkel ; l'arachidyl glucoside, par exemple sous la forme du mélange d'alcools arachidique et béhénique et d'arachidyl glucoside commercialisé sous la dénomination MONTANOV 202 par la société Seppic ; le cocoyléthylglucoside, par exemple sous la forme du mélange (35/65) avec les alcools cétylique et stéarylique, commercialisé sous la dénomination MONTANOV 82 par la société Seppic ; et leurs mélanges.

Quand la composition se présente sous forme d'une émulsion, la nature de la phase huileuse de l'émulsion n'est pas critique. La phase huileuse peut ainsi être constituée par tous les corps gras et notamment les huiles, classiquement utilisés dans les domaines cosmétique ou dermatologique. La phase huileuse comprend généralement au moins une huile.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R^{a}COOR^{b} et R^{a}OR^{b} dans laquelle R^{a} représente le reste d'un acide gras comportant de 8 à 29 atomes de carbone, et R^{b} représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures substantiellement linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam®;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoxylés et notamment éthoxylés tels que l'oleth-12, le ceteareth-12 et le ceteareth-20 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1® " et "FLUTEC PC3® " par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050® " et "PF 5060® " par la Société 3M, ou encore le bromoperfluorooctyle vendu sous la dénomination "FORALKYL® " par la Société Atochem ; le nonafluorométhoxybutane vendu sous la dénomination "MSX 4518® " par la Société 3M et le nonafluoroéthoxyisobutane ; les dérivés de perfluoromorpholine, tels que la 4-tritluorométhyl perfluoromorpholine vendue sous la dénomination "PF 5052® " par la Société 3M ;
- les huiles de silicone comme les polyméthylsiloxanes (PDMS) volatiles ou non à chaîne siliconée substantiellement linéaire ou cyclique, liquides ou pâteux à température ambiante, notamment les cyclopolydiméthylsiloxanes (cyclométhicones) telles que la cyclohexadiméthylsiloxane et la cyclopentadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; la pâte de vaseline ; les gommes telles que les gommes de silicone (diméthiconol) ; les résines de silicone telles que la trifluorométhyl-C1-4-alkyldimethicone et la trifluoropropyldimethicone.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition de l'invention contient au moins une huile de silicone, de préférence une huile de silicone volatile qui peut être choisie par exemple parmi les polydiméthylsiloxanes cycliques ou substantiellement linéaires, et leurs mélanges. Les polydiméthylsiloxanes cycliques ou cyclométhicones comportent d'environ 3 à 9 atomes de silicium, et de préférence de 4 à 6 atomes de silicium et peuvent être par exemple le cyclohexadiméthyl-siloxane et le cyclopentadiméthylsiloxane. Les polydiméthylsiloxanes substantiellement linéaires volatiles comportent de préférence d'environ 3 à 9 atomes de silicium. Les polydiméthylsiloxanes substantiellement linéaires volatiles ont généralement une viscosité à 25°C inférieure ou égale à 5 cSt tandis que les cyclométhicones ont généralement une viscosité à 25°C inférieure ou égale à 10 cSt.

De façon connue, toutes les compositions de l'invention peuvent contenir un ou plusieurs des adjuvants habituels dans les domaines cosmétique et dermatologique, des agents gélifiants et/ou épaississants hydrophiles ou lipophiles ; des agents hydratants ; des émollients ; des actifs hydrophiles ou lipophiles ; des agents anti-radicaux libres ; des séquestrants ; des antioxydants ; des conservateurs ; des agents alcanisants ou acidifiants ; des parfums ; des agents filmogènes ; des matières colorantes (pigments tels que les oxydes de fer et le dioxyde de titane), nacres, colorants solubles), des charges minérales ; et leurs mélanges.

Les quantités de ces différents adjuvants sont celles classiquement utilisées dans les domaines considérés. En particulier, les quantités d'actifs varient selon le but recherché et sont celles classiquement utilisées dans les domaines considérés, et par exemple de 0,1 à 20 %, et de préférence de 0,5 à 10 % du poids total de la composition.

Comme gélifiants hydrophiles autres que les polymères décrits ci-dessus, on peut citer par exemple les polymères carboxyvinyliques tels que les carbopols (carbomers) et les Pemulen (Copolymère acrylate/C₁₀-C₃₀-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les dérivés cellulosiques tels que l'hydroxyéthylcellulose ; les polysaccharides et notamment les gommes telles que la gomme de xanthane ; et leurs mélanges.

Comme gélifiants lipophiles, on peut citer les argiles modifiées telles que le l'hectorite et ses dérivés, comme les produits commercialisés sous les noms de Bentone.

### Actifs

Comme indiqué ci-dessus, la composition de l'invention est stable en présence d'actifs hydrophiles sensibles à l'oxydation et permet de stabiliser de tels actifs. Selon l'invention, on entend par « actif hydrophile », un composé ayant une solubilité dans l'eau d'au moins 0,25 % à température ambiante (25°C). En outre, selon l'invention, on entend par « actif hydrophile sensible à l'oxydation » tout actif d'origine naturelle ou synthétique susceptible de subir une dégradation par un mécanisme d'oxydation. Ce phénomène d'oxydation peut avoir plusieurs causes, en particulier la présence d'oxygène, de lumière, d'ions métalliques, une température élevée, ou encore certaines conditions de pH.

On peut citer à titre d'exemple d'actif hydrophile sensible à l'oxydation, et de façon non limitative, l'acide ascorbique et ses dérivés tels que le 5,6-di-O-diméthylsilylascorbate (vendu par la Sté Exsymol sous la référence PRO-AA), le sel de potassium du di-alpha-tocopheryl-2l-ascorbyl-phosphate (vendu par la Société Senju Pharmaceutical sous la référence SEPIVITAL EPC), l'ascorbyl phosphate de magnésium, l'ascorbyl phosphate de sodium (vendu par la Société Roche sous la référence Stay-C 50) ; le phloroglucinol ; les enzymes ; et leurs mélanges. Parmi les actifs hydrophiles sensibles à l'oxydation, on utilise selon un mode de réalisation préféré de l'invention l'acide ascorbique. L'acide ascorbique peut être de toute nature. Ainsi, il peut être d'origine naturelle sous forme de poudre ou sous forme de jus d'orange de préférence concentré. Il peut être aussi d'origine synthétique, de préférence sous forme de poudre.

Comme autres actifs utilisables dans la composition de l'invention, on peut citer par exemple les agents hydratants tels que les hydrolysats de protéines et les polyols comme la glycérine, les glycols comme les polyéthylène glycols, et les dérivés de sucre ; les extraits naturels ; les anti-inflammatoires ; les oligomères procyannidoliques , les vitamines comme la vitamine A (rétinol), la vitamine E (tocophérol), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; les filtres solaires ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents matifiants comme les fibres ; les agents tenseurs ; et leurs mélanges.

Comme exemples de stéroïdes, on peut citer la déhydroépiandrostérone (ou DHEA), ainsi que (1) ses précurseurs et dérivés biologiques, en particulier les sels et esters de DHEA, tels que le sulfate et le salicylate de DHEA, la 7-hydroxy DHEA, la 7-céto DHEA, les esters de 7-hydroxy et 7-céto DHEA, notamment la 3-beta-acétoxy-7-oxo DHEA, et (2) ses précurseurs et dérivés chimiques, en particulier les sapogénines telles que la diosgénine ou l'hécogénine, et/ou leurs dérivés tels que l'acétate d'hécogénine, et/ou les extraits naturels en contenant et notamment les extraits de Dioscorées, tels que l'igname sauvage (Wild Yam).

Les filtres solaires (ou filtres U.V.) peuvent être choisis parmi les filtres organiques, les filtres physiques et leurs mélanges.

Comme filtres solaires chimiques utilisables dans la composition de l'invention, la composition de l'invention peut comprendre tous les filtres UVA et UVB utilisables dans le domaine cosmétique.

Comme filtres UVB, on peut citer par exemple :
(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, commercialisé par la société Givaudan sous la dénomination Parsol MCX ;
(3) les dérivés de β,β-diphénylacrylate liquides, en particulier l'Octocrylène (α-cyano-β,β diphénylacrylate de 2-éthylhexyle) commercialisé par la société BASF sous la dénomination UVINUL N539 ; et l'Etocrylene, vendu notamment sous le nom commercial « UVINUL N35 » par BASF ;
(4) les dérivés de l'acide p-aminobenzoïque tels que l'Ethylhexyl Diméthyl PABA vendu notamment sous le nom « ESCALOL 507 » par ISP, le Glyceryl PABA, et le PEG-25 PABA vendu sous le nom « UVINUL P25 » par BASF ;
(5) le 4-méthyl benzylidène camphre commercialisé par la société Merck sous la dénomination EUSOLEX 6300 ;
(6) l'acide 2-phénylbenzimidazole 5-sulfonique commercialisé sous la dénomination EUSOLEX 232 par la société Merck ;
(7) les dérivés de 1,3,5-triazine, en particulier :
   - la 2,4,6-tris[p-(2'-éthylhexyl-1 '-oxycarbonyl)anilino]-1 ,3,5-triazine commercialisé par la société BASF sous la dénomination UVINUL T150, et
   - le dioctyl butamido triazone commercialisé par la société Sigma 3V sous la dénomination UVASORB HEB ;
(8) les mélanges de ces filtres.

Comme filtres UVA, on peut citer par exemple :
(1) les dérivés de dibenzoylméthane, en particulier le 4-(ter.-butyl) 4'-méthoxy dibenzoylméthane commercialisé par la société Givaudan sous la dénomination PARSOL 1789 ;
(2) l'acide benzène 1,4 [di(3-méthylidènecampho-10-sulfonique)] éventuellement sous forme partiellement ou totalement neutralisée, commercialisé sous la dénomination MEXORYL SX par la société Chimex.
(3) les dérivés de benzophénone, par exemple :
   - la 2,4-dihydroxybenzophénone (benzophénone-1) ;
   - la 2,2',4,4'-tétra-hydroxybenzophénone (benzophénone-2) ;
   - la 2-hydroxy-4-méthoxy-benzophénone (benzophénone-3), commercialisé sous la dénomination UVINUL M40 par la société BASF ;
   - l'acide, 2-hydroxy-4-méthoxy-benzophénone-5-sulfonique (benzophénone-4) ainsi que sa forme sulfonate (benzophénone-5), commercialisé par la société BASF sous la dénomination UVINUL MS40 ;
   - la 2,2'-dihydroxy-4,4'-diméthoxy-benzophénone (benzophénone-6);
   - la 5-chloro-2-hydroxybenzophénone (benzophénone-7) ;
   - la 2,2'-dihydroxy-4-méthoxy-benzophénone (benzophénone-8) ;
   - le sel disodique du diacide 2,2'-dihydroxy-4,4'-diméthoxy-benzophénone-5,5'-disulfonique (benzophénone-9) ;
   - la 2-hydroxy-4-méthoxy-4'-méthyl-benzophénone (benzophénone-10) ;
   - la benzophénone-11 ;
   - la 2-hydroxy-4-(octyloxy)benzophénone (benzophénone-12).
(4) les dérivés silanes ou les polyorganosiloxanes à groupement bénzophénone ;
(5) les anthranilates, en particulier l'anthranilate de menthyle commercialisé par la société Haarman & Reiner sous la dénomination NEO HELIOPAN MA ;
(6) les composés comportant par molécule au moins deux groupes benzoazolyle ou au moins un groupe benzodiazolyle, en particulier l'acide 1,4-bis-benzimidazolyl-phenylèn-3,3',5,5'-tétrasulfonique ainsi que ses sels commercialisés par la société Haarman & Reimer ;
(7) les dérivés siliciés de benzimidazolyl-benzazoles N-substitués ou de benzofuranyl-benzazoles, et en particulier :
   - le 2-[1-[3-[1,3,3,3-tétraméthyl-1-[(triméthylsilyl)oxy]disiloxanyl]propyl]-1H-benzimidazol-2-yl]-benzoxazole ;
   - le 2-[1-[3-[1,3,3,3-tétraméthyl-1-[(triméthylsilyl)oxy]disiloxanyl]propyl]-1H-benzimidazol-2-yl]-benzothiazole ;
   - le 2-[1-(3-trimethylsilanyl-propyl)-1H-benzimidazol-2-yl]-benzoxazole ;
   - le 6-méthoxy-1,1'-bis-(3-triméthylsilanyl-propyl)-1H,1'H-[2,2']bibenzimidazolyl-benzoxazole ;
   - le 2-[1-(3-trimethylsilanyl-propyl)-1H-benzimidazol-2-yl]-benzothiazole ;
   qui sont décrits dans la demande de brevet EP-A-1 028 120 ;
(8) les dérivés de triazine, et en particulier la 2,4-bis {[4-(2-éthyl-hexyloxy)-2-hydroxy]-phényl}-6-(4-méthoxy-phényl)-1,3,5-triazine commercialisé par la société Ciba Geigy sous la dénomination TINOSORB S, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M ;
(9) leurs mélanges.

On peut aussi utiliser un mélange de plusieurs de ces filtres et un mélange de filtres UVB et de filtres UVA et aussi des mélanges avec des filtres physiques.

Comme filtres physiques, on peut citer les oxydes de titane (dioxyde de titane amorphe ou cristallisé sous forme rutile et/ou anatase), de zinc, de fer, de zirconium, de cérium ou leurs mélanges. Ces oxydes métalliques peuvent être sous forme de particules ayant une taille micrométrique ou nanométrique (nano-pigments). Sous forme de nano-pigments, les tailles moyennes des particules vont par exemple de 5 à 100 nm.

Par ailleurs, il est particulièrement avantageux d'introduire au moins une charge minérale dans la composition selon l'invention, notamment quand la composition contient au moins une poudre organique, afin d'obtenir une composition particulièrement confortable lors de l'application sur la peau, c'est-à-dire à la fois souple, douce, émolliente et hydratante.

### Charges minérales

Les charges minérales pouvant être utilisées dans la composition de l'invention peuvent être très variées. Elles peuvent être choisies notamment parmi le talc ; le kaolin ; le nitrure de bore ; les oxydes métalliques (qui se trouvent aussi dans les filtres physiques décrits ci-dessus) ; les micas ; les nacres ; les poudres de silice (ou dioxyde de silicium) ; l'oxychlorure de bismuth ; le stéarate de zinc ; les particules de sel de métal alcalin ou alcalinoterreux comme les particules de carbonate de calcium, de sulfate de baryum, de sulfate de calcium ; les particules de platine ; les alumines (notamment les alumines actives) ; les alumino-silicates (argiles notamment) ; les silicates mixtes de métaux alcalins et/ou alcalino-terreux (smectites, Laponites, en particulier LAPONITES DS, D, XLS ou XLG commercialisées par la Société LAPORTE Industries, Ltd.) ; les zéolithes ; la magnésie ; les matériaux composites à base de charges minérales ; et leurs mélanges.

Comme oxydes métalliques, on peut citer par exemple les oxydes de titane (dioxyde de titane amorphe ou cristallisé sous forme rutile et/ou anatase), de zinc, de fer, de zirconium, de cérium ou leurs mélanges. Ces oxydes métalliques peuvent être sous forme de particules ayant une taille micrométrique ou nanométrique ayant une taille moyenne de particules allant par exemple de 5 à 100 nm. Ces oxydes métalliques peuvent être enrobés et notamment avoir un enrobage hydrophobe. Les oxydes métalliques enrobés pouvant être utilisés dans la composition de l'invention peuvent par exemple avoir subi un ou plusieurs traitements avec un ou plusieurs composés choisis parmi l'alumine, la silice, les dérivés de l'aluminium (par exemple stéarate et laurate), les composés du silicium (par exemple silicones, polydiméthylsiloxanes, alcoxysilanes, siloxysilicates), les composés du sodium, les oxydes de fer, les esters de fer (par exemple stéarate), les acides gras, les alcools gras et leurs dérivés (tels que l'acide stéarique, l'acide hydroxystéarique, l'alcool stéarylique, l'alcool hydroxystéarylique, l'acide laurique et leurs dérivés), la lécithine, les cires (par exemple la cire de carnauba), les polymères (méth)acryliques (par exemple les polyméthylmethacrylates), les composés fluorés (par exemple les composés perfluoroalkyle et les perfluoroalkyle éthers). Les oxydes peuvent aussi être traités par un mélange de ces composés ou ils peuvent comprendre plusieurs enrobages successifs.

On peut aussi utiliser des nacres. Par nacres, il faut comprendre des particules irisées qui réfléchissent la lumière. Parmi les nacres envisageables, on peut citer la nacre naturelle, le mica recouvert d'oxyde de titane, d'oxyde de fer, d'hydroxyde d'aluminium, d'hydroxyde de magnésium, de silice, de pigment naturel et d'oxychlorure de bismuth, ainsi que le mica titane coloré, et leurs mélanges.

La silice pouvant être utilisée comme charge minérale peut être choisie parmi les microsphères de silice, comme celles commercialisées sous la dénomination SUNSPHERE H-51 par la société Asahi Glass, ou sous la dénomination Silica Beads par la Société MAPRECOS.

On peut aussi utiliser les silices hydrophiles ou hydrophobes commercialisées par la société Degussa-Hüls sous les dénominations AEROSIL 90, AEROSIL 130, AEROSIL 150, AEROSIL 200, AEROSIL300, AEROSIL380, AEROSIL OX 50, SILICE FK 320 DS, AEROSIL R202, AEROSIL R805, AEROSIL R812, AEROSIL R972, AEROSIL R974.

On peut encore utiliser de la silice en dispersion aqueuse, et par exemple une dispersion de silice colloïdale, telle que le produit commercialisé sous la dénomination BINDZIL 30/220® par la société Eka Chemicals, dispersion colloïdale de silice amorphe (taille : 14 nanomètres) dans l'eau (30/70), ou le produit commercialisé sous la dénomination COSMO S-40 par la société Catalysts Chemicals (taille : 18 nanomètres).

La silice peut consister également en une particule recouverte totalement ou partiellement de silice, notamment d'une particule minérale recouverte totalement ou partiellement de silice, telle que les billes de silice contenant de l'oxyde de titane, commercialisées sous la dénomination TORAYCERAM S-IT® par la société Toray ; les microsphères de silice-alumine contenant de l'oxyde de titane (taille : 105 µm), commercialisées sous la dénomination Z-LIGHT-SPHERE W 1012® par la société Zeelan ; les particules de silice synthétique précipitée amorphe/oxyde de titane (taille : 106-500 µm), commercialisées sous la dénomination NEOSIL PC20S® par la société Crosfield ; les fibres de Nylon-6 - silice - oxyde de titane (longueur de 2 mm et épaisseur de 2 deniers), commercialisées sous la dénomination FIBERLON Y2® par la société Wackherr ; la silice enrobée de dioxyde de titane et recouvert de silice poreuse (85/5/10) (taille : 0,6 µm), commercialisée sous la dénomination ACS-0050510® par la société SACI-CFPA ; le nano-oxyde de titane anatase traité alumine et silice à 40% dans l'eau (taille : 60 nm, monodisperse), commercialisé sous la dénomination MIRASUN TIW 60® par la société Rhodia Chimie CRA ; le nano-oxyde de titane anatase (60 nm) enrobé silice/alumine/cerium IV 15/5/3 en dispersion aqueuse à 32 %, commercialisé sous la dénomination MIRASUN TIW 160® par la société Rhodia Chimie CRA ; le nano-oxyde de titane anatase traité alumine et silice (34/4,3/1,7) en dispersion aqueuse à 40 %, commercialisé sous la dénomination TIOVEIL AQ-N® par la société Uniqema ; le nano-oxyde de titane enrobé de silice (66/33) (granulométrie du dioxyde de titane : 30 nm ; épaisseur de silice : 4 nm), commercialisé sous la dénomination MAXLIGHT TS-04® par la société Nichimen Europe PLC ; et le nano-oxyde de titane enrobé de silice (80/20) (granulométrie dioxyde de titane : 30 nm ; épaisseur de silice : 2 nm) commercialisé sous la dénomination MAXLIGHT TS-042® par la société Nichimen Europe PLC. Ces particules peuvent également avoir des propriétés optiques dans la composition comme sur la peau.

Selon les cas, la charge minérale peut être introduite dans la composition après mélange des autres constituants et, par exemple, dans le cas d'une émulsion après préparation de l'émulsion, ou bien, si une phase huileuse est présente, dans la phase huileuse de la composition.

Les charges minérales pouvant être utilisées dans la composition conforme à l'invention sont généralement présentes dans des quantités en matière active allant de 0,05 à 30 % en poids, plus préférentiellement de 0,1 à 20 % en poids, encore plus préférentiellement de 0,5 à 10 % en poids par rapport au poids total de la composition.

Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.

La composition présente de préférence un pH qui respecte la peau et qui va généralement de 3 à 8 et de préférence de 4,5 à 7.

Les compositions de l'invention peuvent être utilisées comme produit de soin (ou de traitement), de protection, de nettoyage, de démaquillage et/ou de maquillage des matières kératiniques (peau, cheveux, cuir chevelu, cils, sourcils, ongles ou muqueuses) tels que des crèmes de protection, de traitement ou de soin pour le visage, pour les mains ou pour le corps, des laits corporels de protection ou de soin, des gels ou mousses pour le soin de la peau et/ou des muqueuses (lèvres).

Les compositions de l'invention contenant des filtres solaires peuvent être également utilisées comme produit de protection solaire.

Les compositions de l'invention peuvent aussi être utilisées comme produits pour le maquillage, notamment le maquillage de la peau, des sourcils, des cils et des lèvres. Les produits de maquillage sont le plus souvent colorés et contiennent généralement des pigments. Sous forme de produits de maquillage, les compositions de l'invention peuvent constituer avantageusement un fond de teint, un rouge à lèvres, un fard à joues, un fard à paupières, un mascara ou un eyeliner.

Les compositions selon l'invention peuvent être également utilisées comme produits rincés ou comme produits non-rincés pour le nettoyage de la peau du visage et/ou du corps et/ou pour le nettoyage des cheveux, par exemple comme produits capillaires y compris pour le soin et le conditionnement des cheveux.

L'invention a aussi pour objet l'utilisation cosmétique d'une composition cosmétique telle que définie ci-dessus, comme produit de soin, de nettoyage et/ou de démaquillage de la peau, des cheveux, du cuir chevelu, des cils, des sourcils, des ongles ou des muqueuses.

L'invention a aussi pour objet l'utilisation cosmétique d'une composition cosmétique telle que définie ci-dessus, comme produit de maquillage.

L'invention a aussi pour objet l'utilisation cosmétique d'une composition cosmétique telle que définie ci-dessus, comme produit de protection solaire (protection contre le soleil et/ou les U.V. des appareils à bronzer).

L'invention a aussi pour objet l'utilisation cosmétique d'une composition cosmétique telle que définie ci-dessus, comme produit capillaire rincé ou non-rincé.

Un autre objet de l'invention est un procédé de traitement cosmétique (non-thérapeutique) d'une matière kératinique (peau, cuir chevelu, cheveux, cils, sourcils, ongles ou muqueuses), caractérisé en ce qu'on applique sur la matière kératinique, une composition cosmétique telle que définie ci-dessus. La matière kératinique est notamment la peau.

Les exemples qui suivent permettront de mieux comprendre l'invention, sans toutefois présenter un caractère limitatif. Les quantités indiquées sont en % en poids, sauf mention contraire.

### Exemple 1 selon l'invention : Gel

| | |
|---|---|
| Ammonium polyacryldimethyl tauramide (Hostacerin AMPS de la société Clariant) | 1 % |
| Conservateur | qs % |
| HMW2220 (Dow Corning) | 2 % |
| Eau | qsp 100 % |

Mode opératoire : On prépare le gel d'AMPS à chaud sous agitation. On refroidit puis on ajoute sous faible cisaillement de la dispersion, la dispersion de HMW2220.

On obtient un gel translucide, très léger, doux et frais à l'application sans toucher gras.

### Exemple 1 comparatif : Gel

| | |
|---|---|
| Sodium Carbomer (Gélifiant) | 1 % |
| Conservateur | qs % |
| HMW2220 (Dow Corning) (dispersion aqueuse à 60 % de M.A.) | 2 % |
| (soit 1,2 % de M.A.) | |
| Eau | qsp 100 % |

Mode opératoire : On prépare le gel de carbomer à chaud sous agitation. On refroidit puis on ajoute sous faible cisaillement de la dispersion, la dispersion de HMW2220.

On obtient un gel blanc à translucide, doux à l'application mais collant pendant l'application et laissant un aspect collant en fin d'application.

### Exemple 2 selon l'invention : Emulsion H/E

| *Phase A (phase huileuse)* | |
|---|---|
| Mélange de cocoyl éthylglucoside et d'alcool cetylique, stéarylique (35/65) (Montanov 82 de la société SEPPIC) | 3 % |
| Huile d'abricot | 10 % |
| Cyclohexadiméthylsiloxane | 5 % |
| Pâte de vaseline | 10 % |
| Acide stéarique | 0,5 % |

| *Phase B (phase aqueuse)* | |
|---|---|
| Glycérine | 5 % |
| Copolymère acrylamide/acrylamido-2-methyl propane sulfonate de sodium en émulsion inverse à 40% dans le polysorbate (Simulgel 600 de la société Seppic) | 1 % |
| Conservateur | qs % |
| Eau | qsp 100 % |

| *Phase C* | |
|---|---|
| HMW2220 (Dow Corning) (dispersion aqueuse à 60 % de M.A.) (soit 1,2 % de M.A.) | 2 % |
| Parfum | 0,1 % |

Mode opératoire : On prépare la phase B à chaud sous agitation. On y ajoute la phase A. On refroidit puis on ajoute sous faible cisaillement de la dispersion, la phase C.

On obtient une crème blanche, très douce à l'application sans toucher gras ni collant, donnant une bonne sensation d'hydratation après application.

### Exemple 3 selon l'invention : Fond de teint (H/E)

| *Phase A (phase huileuse)* | |
|---|---|
| Cyclopentadiméthylsiloxane | 4 % |
| Cyclohexadiméthylsiloxane | 10 % |
| Huiles de silicone non volatiles | 12 % |
| (polyphénylméthylsiloxane et polycétylméthylsiloxane) | |
| Stéarate de polyéthylène glycol | 0,6 % |
| Stéarate de glycérol | 0,3 % |
| Acide stéarique | 1,9 % |
| Oxyde de fer jaune enrobé | 1,6 % |
| Oxyde de fer brun | 0,5 % |
| Oxyde de fer noir | 0,3 % |
| Poudre de nylon 12 | 6 % |
| Poudre de polyéthylène | 4 % |

| *Phase B (phase aqueuse)* | |
|---|---|
| Propylène glycol | 6,5 % |
| Polyéthylène glycol 32 OE (PEG-32) | 10 % |
| Conservateur | qs |
| Ammonium polyacryldimethyl tauramide (Hostacerin AMPS de la société Clariant) | 1,5 % |
| Eau | qsp |

| *Phase* C | |
|---|---|
| HMW2220 (Dow Corning) (dispersion aqueuse à 60 % de M.A.) (soit 1,2 % de M.A.) | 2 % |
| Parfum | 0,1 % |

Mode opératoire : On prépare la composition en chauffant les constituants de la phase huileuse A, hormis les huiles volatiles, à 65°C et en mélangeant. On ajoute ensuite, à 60°C, les huiles volatiles. Parallèlement, on prépare la phase aqueuse B à 80°C. On laisse refroidir jusqu'à 30°C. On mélange sous agitation Moritz les deux phases en versant la phase huileuse dans la phase aqueuse. Ensuite, on ajoute la phase C sous faible agitation.

On obtient ainsi un fond de teint doux. Ce fond de teint est fluide, coloré et de texture très douce ; il s'étale bien et s'applique uniformément.

### Exemple 4 selon l'invention : Emulsion H/E

| *Phase A (phase huileuse)* | |
|---|---|
| Cyclohexadiméthylsiloxane | 5 % |
| Isohexadécane | 5 % |

| *Phase B (phase aqueuse)* | |
|---|---|
| Glycérine | 4 % |
| Propylène glycol | 3 % |
| Arlacel 165 (Glyceryl stearate/PEG-5 stearate) | 2 % |
| Conservateur | qs % |
| Ammonium polyacryldimethyl tauramide (Hostacerin AMPS de la société Clariant | 1 % |
| Eau | qsp 100 % |

| Phase C | |
|---|---|
| Amidon modifié (Aluminium Starch Octenyl succinate DRY FLO de la société National Starch) | 2 % |
| Acrylates copolymer (Expancel 551 DE20 de la société Kemanord Plast) | 0,5 % |

| *Phase D* | |
|---|---|
| HMW2220 (Dow Corning) (dispersion aqueuse à 60 % de M.A.) (soit 1,2 % de M.A.) | 2 % |

Mode opératoire : On prépare les phases A et B et on incorpore la phase A dans la phase B sous agitation. On y ajoute la phase C. Puis on ajoute sous faible cisaillement, la phase D.

On obtient une crème homogène blanche, poudrée, douce à l'application et facile à étaler.

### Exemple 5 selon l'invention : Emulsion H/E

| *Phase A (phase huileuse)* | |
|---|---|
| Mélange d'arachidyl polyglucoside et d'alcools arachidique et béhénique (15/85) (Montanov 202 de la société SEPPIC) | 1,5% |
| Mélange de mono-di-stéarate de glycéryle et de stéarate de potassium (TEGIN de la société Goldschmidt) | 1,5% |
| Huile d'abricot | 5 % |
| Cyclohexadiméthylsiloxane - | 10 % |

| *Phase B (phase aqueuse)* | |
|---|---|
| Glycérine | 5 % |
| Ammonium polyacryldimethyl tauramide (Hostacerin AMPS de la société Clariant) | 1 % |
| Conservateur | qs |
| Eau | qsp 100 % |

| *Phase C* | |
|---|---|
| Poudre de Nylon (ORGASOL 2002 D NAT COS) | 2 % |

| *Phase D* | |
|---|---|
| HMW2220 (Dow Corning) (dispersion aqueuse à 60 % de M.A.) (soit 1,2 % de M.A.) | 2 % |

Mode opératoire : On prépare les phases A et B à chaud (environ 80°C) et on incorpore la phase A dans la phase B sous agitation. Après refroidissement à 40°C, on ajoute la phase C. Puis on ajoute sous faible cisaillement, la phase D.

On obtient une crème homogène blanche, poudrée, douce à l'application et facile à étaler.

### Exemple 6 selon l'invention : Emulsion E/H

| *Phase A (phase huileuse)* | |
|---|---|
| Cetyl dimethicone copolyol | |
| (ABIL EM 90 de la société Goldschmidt) | 1,5 % |
| Isostéarate de polyglycérole | |
| (Isolan GI 34 de la société Goldschmidt) | 0,5 % |
| Isohexadécane | 10 % |
| Huile d'abricot | 5 % |
| Cyclohexadiméthylsiloxane | 8 % |
| Acrylates copolymer (Expancel 551 DE20 d'Expancel) | 0,5 % |
| Poudre de polydiméthylsilsesquioxane | |
| (Tospearl 240 de la société Toshiba) | 1 % |

| *Phase B (phase aqueuse)* | |
|---|---|
| Glycérine | 5 % |
| Sulfate de magnésium | 0,7 % |
| HMW2220 (Dow Corning) (dispersion aqueuse à 60 % de M.A.) (soit 1,2 % de M.A.) | 2 % |
| Conservateur | qs |
| Eau | qsp 100 % |

Mode opératoire : On prépare les phases A et B à froid et on incorpore la phase B dans la phase A sous agitation.

On obtient une crème homogène blanche souple, qui n'est pas grasse quand on l'applique sur la peau.

### Exemple 7 selon l'invention : Gel nacré

| *Phase A* | |
|---|---|
| Glycérine | 4 % |
| Propylène glycol | 3 % |
| Conservateur | qs % |
| Copolymère acrylamide/acrylamido-2-methyl propane sulfonate de sodium en émulsion inverse à 40% dans le polysorbate | |
| (Simulgel 600 de la société SEPPIC) | 1,5 % |
| Poudre de copolymère méthacrylate de méthyle / diméthacrylate | |
| d'éthylène glycol (Microsphères M305 de la société Matsumoto) | 1 % |
| Mica-oxyde de titane (Flamenco red 420C de le société Engelhard) | 2 % |
| Eau | qsp 100 % |

| *Phase B* | |
|---|---|
| HMW2220 (Dow Corning) (dispersion aqueuse à 60 % de M.A.) (soit 1,2 % de M.A.) | 2 % |

Mode opératoire : On mélange les ingrédients de la phase A à chaud sous agitation. Ensuite, on refroidit à 30°C et on ajoute la phase B sous faible cisaillement.

On obtient un gel, translucide et nacré, très doux au toucher et frais lors de l'application sur la peau. Ce gel peut s'utiliser en tant que crème corps ou visage.

### Exemple 8 selon l'invention : Emulsion H/E

| *Phase A (phase huileuse)* | |
|---|---|
| Mélange d'arachidyl polyglucoside et d'alcools arachidique et béhénique (15/85) (Montanov 202 de la société SEPPIC) | 1,5 % |
| Mélange de mono-di-stéarate de glycéryle et de stéarate de Potassium (93/7) (TEGIN de la société Goldschmidt) | 1,5 % |
| Cyclohexadiméthylsiloxane | 5 % |

| *Phase B (phase aqueuse)* | |
|---|---|
| Glycérine | 5 % |
| Conservateur | qs |
| Eau | qsp 100 % |

| *Phase* C | |
|---|---|
| Ammonium polyacryldimethyl tauramide (Hostacerin AMPS) | 1 % |
| Gomme de xanthane | 0,2 % |
| Cyclohexadiméthylsiloxane | 5 % |

| *Phase D* | |
|---|---|
| Acide ascorbique | 5 % |
| Eau | 20 % |

| *Phase E* | |
|---|---|
| HMW2220 (Dow Corning) (dispersion aqueuse à 60 % de M.A.) (soit 1,8 % de M.A.) | 3 % |

Mode opératoire : A 70-75°C, on homogénéise la phase A sous agitateur magnétique. A 85°C, on homogénéise la phase B sous agitateur magnétique. A 70-75°C, sous agitation, on introduit la phase A dans la phase B. Puis, on introduit la phase C dans le mélange obtenu. On mélange jusqu'à l'obtention d'une émulsion fine et on refroidit. A 25°C, on introduit la phase D et ensuite la phase E, sous agitation douce.

On obtient une crème blanche, glissante, très douce à l'application.

Après trois semaines à 50°C, l'émulsion est restée stable et elle ne présente pas de relargage ni de séparation de phases. En outre, l'évolution de la couleur (jaunissement) est beaucoup moins important qu'avec les compositions de l'art antérieur (voir ci-dessous exemples comparatifs).

### Exemple 2 comparatif : Emulsion H/E

| *Phase A (phase huileuse)* | |
|---|---|
| Mélange d'arachidyl polyglucoside et d'alcools arachidique et béhénique (15/85) (Montanov 202 de la société SEPPIC) | 1,5 % |
| Mélange de mono-di-stéarate de glycéryle et de stéarate de Potassium (93/7) (TEGIN de la société Goldschmidt) | 1,5 % |
| Cyclohexadiméthylsiloxane | 5 % |

| *Phase B (phase aqueuse)* | |
|---|---|
| Glycérine | 5 % |
| Conservateur | qs |
| Eau | qsp 100 % |

| *Phase C* | |
|---|---|
| Ammonium polyacryldimethyl tauramide (Hostacerin AMPS) | 1 % |
| Gomme de xanthane | 0,2 % |
| Cyclohexadiméthylsiloxane | 5 % |

| *Phase D* | |
|---|---|
| Acide ascorbique | 5 % |
| Eau | 20 % |

Mode opératoire : A 70-75°C, on homogénéise la phase A sous agitateur magnétique. A 85°C, on homogénéise la phase B sous agitateur magnétique. A 70-75°C, sous agitation, on introduit la phase A dans la phase B. Puis, on introduit la phase C dans le mélange obtenu. On mélange jusqu'à l'obtention d'une émulsion fine et on refroidit. A 25°C, on introduit la phase D sous agitation douce.

On obtient une crème blanche, glissante, rêche à l'application.

Après trois semaines à 50°C, l'émulsion est déstabilisée et il y a relargage d'huile et d'eau au fond du pot contenant l'émulsion. En outre, la composition a fortement jauni.

### Exemple 3 comparatif : Emulsion H/E

| *Phase A (phase huileuse)* | |
|---|---|
| Mélange d'arachidyl polyglucoside et d'alcools arachidique et béhénique (15/85) (Montanov 202 de la société SEPPIC) | 1,5 % |
| Mélange de mono-di-stéarate de glycéryle et de stéarate de Potassium (93/7) (TEGIN de la société Goldschmidt) | 1,5 % |
| Cyclohexadiméthylsiloxane | 3 % |
| Elastomère siliconé en dispersion huileuse (mélange de Polydiméthylsiloxane réticulé et de polydiméthylsiloxane (6 cst) (24/76) (KSG 16) | 3 % |

| *Phase B (phase aqueuse)* | |
|---|---|
| Glycérine | 5 % |
| Conservateur | qs |
| Eau | qsp 100 % |

| *Phase C* | |
|---|---|
| Ammonium polyacryldimethyl tauramide (Hostacerin AMPS) | 1 % |
| Gomme de xanthane | 0,2 % |
| Cyclohexadiméthylsiloxane | 5 % |

| *Phase D* | |
|---|---|
| Acide ascorbique | 5 % |
| Eau | 20 % |

Mode opératoire : A 70-75°C, on homogénéise la phase A sous agitateur magnétique. A 85°C, on homogénéise la phase B sous agitateur magnétique. A 70-75°C, sous agitation, on introduit la phase A dans la phase B. Puis, on introduit la phase C dans le mélange obtenu. On mélange jusqu'à l'obtention d'une émulsion fine et on refroidit. A 25°C, on introduit la phase D sous agitation douce.

On obtient une crème blanche, glissante, très douce à l'application.

Après trois semaines à 50°C, l'émulsion est déstabilisée et il y a un relargage très important d'huile et un relargage important d'eau au fond du pot contenant l'émulsion. En outre, la composition a fortement jauni.

Il ressort de l'exemple 8 selon l'invention et des exemples comparatifs 2 et 3 ci-dessus que la composition selon l'invention contenant la vitamine C est beaucoup plus stable qu'une composition ne contenant pas de dispersion de copolymère siliconé ou qu'une composition contenant une dispersion huileuse d'élastomère de silicone réticulé de l'art antérieur.

### Exemple 10 selon l'invention : Sérum

| | |
|---|---|
| Ammonium Polyacryldimethyl tauramide (Hostacerin AMPS) | 0,8 % |
| Hyaluronate de sodium | 0,1 % |
| HMW2220 (Dow Corning) (dispersion aqueuse à 60 % de M.A.) (soit 3 % de M.A.) | 5 % |
| Covabead LH85 (Wackherr) | 2 % |
| Dioxyde de titane | 1 % |
| Ethanol | 10 % |
| Glycérine | 5 % |
| Parfum | 0,5 % |
| Conservateur | qs % |
| Eau | qsp 100 % |

Mode opératoire : L'AMPS et le hyaluronate de sodium sont dispersés sous agitation dans la phase aqueuse comprenant l'eau, la glycérine et les conservateurs jusqu'à complète solubilisation. L'HMW 2220, les Covabead LH85 et le dioxyde de titane sont dispersés et homogénéisés dans le mélange précédemment obtenu. Puis on y ajoute le parfum présolubilisé dans l'alcool.

On obtient un sérum frais et doux à l'application, que l'on peut utiliser seul ou sous un produit de soin.

### Exemple 11 selon l'invention : Emulsion H/E

| *Phase A (phase huileuse)* | |
|---|---|
| PEG-20 Methyl glucose sesquistearate | 2 % |
| Huile d'abricot | 4 % |
| Cyclohexadiméthylsiloxane | 5 % |
| Cyclopentadiméthylsiloxane/dimethiconol (DC2-9071 de Dow Corning) (gomme de silicone) | 5 % |
| Vitamine E | 0,25 % |
| Palmitate de rétinol - | 0,1 % |

| *Phase B (phase aqueuse)* | |
|---|---|
| Copolymère AMPS/acrylate de sodium en émulsion inverse | |
| (SIMULGEL EG de la société SEPPIC) | 1,5 % |
| Glycérine | 7 % |
| Hostacerin AMPS | 0,5 % |
| Conservateur | qs |
| Colorant | qs |
| Eau | qsp 100 % |

| *Phase C* | |
|---|---|
| HMW2220 (Dow Corning) (dispersion aqueuse à 60 % de M.A.) (soit 1,2 % de M.A.) | 2 % |
| Silice (Sunsphère H51 de la société Asahi) | 8 % |
| Expancel 551DE20 | 0,5 % |

Mode opératoire : On prépare la phase A en chauffant les constituants de cette phase à 60°C. On prépare la phase B en chauffant le mélange d'eau et de glycérine à 65°C, en y solubilisant conservateurs et. colorant, en ajoutant l'Hostacerin AMPS et en maintenant l'agitation jusqu'à gélification. On prépare l'émulsion en versant la phase A à 60°C dans la phase B à 65°C sous agitation, puis on ajoute le Simulgel EG ; on homogénéise et on refroidit à 30°C. On ajoute ensuite la phase C sous agitation.

On obtient un fluide en émulsion qui a un effet de douceur et de confort surprenant et qui peut s'utiliser matin et/ou soir. Ce fluide a été testé sur un panel de 30 femmes qui l'ont trouvé très doux à l'application, léger et confortable, laissant la peau très douce.

### Exemple 12 selon l'invention : Lotion poudrée

| | |
|---|---|
| HMW2220 (Dow Corning) (dispersion aqueuse à 60 % de M.A.) (soit 1,2 % de M.A.) | 2 % |
| Silice (Sunsphère H51 de la société Asahi) | 5 % |
| Covabead LH85 (de la Société Wackherr) | 1 % |
| Dioxyde de zinc | 1 % |
| Ethanol à 96° | 15 % |
| Glycérine | 3 % |
| Parfum | qs % |
| Eau | qsp 100 % |

Mode opératoire : Dans le mélange d'eau et de glycérine, on ajoute sous agitation HMW 2220, silice, Covabead LH85 et dioxyde de zinc, puis ensuite le mélange de parfum et d'éthanol.

On obtient une lotion poudrée matifiante d'une très grande douceur. Cette lotion s'applique après agitation pour la réhomogénéiser avant utilisation. Elle peut s'utiliser matin et /ou soir avant application du produit de soin habituel.

### Exemple 13 selon l'invention : Emulsion E/H (coulée)

| *Phase A (phase huileuse)* | |
|---|---|
| Abil WE09 | 9 % |
| Phényltriméthicone | 5 % |
| Cyclohexadiméthylsiloxane | 15 % |
| Cire de polyéthylène | 3 % |
| Huile de jojoba hydrogénée | 6 % |
| Nylon 12 (Orgasol 2002 Extra de la société Atochem) | 3 % |

| *Phase B (phase aqueuse)* | |
|---|---|
| Glycérine | 7 % |
| Sulfate de magnésium | 1 % |
| HMW2220 (Dow Corning) (dispersion aqueuse à 60 % de M.A.) (soit 3 % de M.A.) | 5 % |
| Silice (Sunsphère H51 de la société Asahi) | 4 % |
| Conservateur | qs |
| Parfum | qs |
| Eau | qsp 100 % |

Mode opératoire : on homogénéise la phase huileuse (A) à 95°C pour fondre les cires. On chauffe la phase aqueuse (B) à 85°C. Puis, on ajoute sous agitation la phase aqueuse dans la phase huileuse et on refroidit à 75°C. On coule dans des coupelles métalliques.

On obtient un produit compact hydratant très doux. Ce produit, de part sa présentation « produit nomade » (c'est-à-dire que l'on peut emporter avec soi) peut être utilisé au cours de la journée quand la peau en ressent le besoin. Il peut s'appliquer au doigt ou à l'éponge.

### Exemple 14 selon l'invention : poudre

| *Phase A* | |
|---|---|
| Talc | 23 % |
| Mica | 22 % |
| Oxychlorure de bismuth | 8 % |
| Stéarate de Zinc | 3 % |
| Nylon 12 (Orgasol 2002 Extra de la société Atochem | 20 % |
| Dioxyde de titane | 2 % |

| *Phase B* | |
|---|---|
| Oxydes de fer | 15,5 % |

| *Phase C (liant)* | |
|---|---|
| Stéarate d'isocétyle | 3,5 % |
| HMW2220 (Dow Corning) (dispersion aqueuse à 60 % de M.A.) (soit 1,8% de M.A.) | 3 % |

Mode opératoire : Les phases A et B sont mélangées, puis on y ajoute goutte à goutte la phase C pré-mélangée. L'ensemble est ensuite broyé dans un broyeur à broches, puis tamisé. Cette poudre est alors compactée dans des coupelles.

On obtient un poudre compacte très douce que l'on applique à l'éponge ou au pinceau soit directement sur la peau soit sur un fond de teint pour obtenir un maquillage velouté.

## Revendications

1. Composition comprenant dans un milieu physiologiquement acceptable, au moins une phase aqueuse comprenant en dispersion, des particules de copolymère siliconé bloc non réticulé obtenu par extension de chaîne, soit au moins un polymère comportant au moins un monomère à insaturation éthylénique et à groupement sulfonique, soit au moins une poudre organique, soit un mélange d'au moins un polymère comportant au moins un monomère à insaturation éthylénique et à groupement sulfonique et d'au moins une poudre organique.

2. Composition selon la revendication 1, **caractérisée par le fait que** la quantité de copolymère siliconé va de 0,01 à 15 % en poids par rapport au poids total de la composition.

3. Composition selon la revendication 1 ou 2, **caractérisée par le fait que** les particules de copolymère siliconé présentent une taille moyenne en nombre inférieure ou égale à 2 microns.

4. Composition selon l'une quelconque des revendications précédentes, **caractérisée par le fait que** le copolymère siliconé est obtenu par réaction d'extension de chaîne, en présence d'un catalyseur, à partir d'au moins :
- (a) un polysiloxane (i) ayant au moins un groupe réactif et de préférence un ou deux groupes réactifs par molécule ; et
- (b) un composé organosiliconé (ii) qui réagit avec le polysiloxane (i) par réaction d'extension de chaîne.

5. Composition selon la revendication précédente, **caractérisée par le fait que** l'organopolysiloxane (i) est choisi parmi les composés de formule (I) : 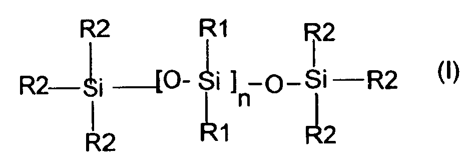 dans laquelle R₁ et R₂ indépendamment les uns des autres représentent un groupe hydrocarboné ayant de 1 à 20 atomes de carbone ou un groupe aryle ou un groupe réactif, n est un nombre entier supérieur à 1, à la condition qu'il y ait en moyenne entre un et deux groupes réactifs par polymère.

6. Composition selon la revendication précédente, **caractérisée par le fait que** le groupe réactif est choisi parmi l'hydrogène ; les groupes aliphatiquement insaturés ; le groupe hydroxyle ; les groupes alcoxy ; les groupes alcoxy-alcoxy ; le groupe acétoxy ; les groupes aminés ; et leurs mélanges.

7. Composition selon la revendication 5 ou 6, **caractérisée par le fait que** R₁ représente un groupe méthyle.

8. Composition selon l'une quelconque des revendications 5 à 7, **caractérisée par le fait que** R₂ en bout de chaîne représente un groupe vinyle.

9. Composition selon la revendication 4, **caractérisée par le fait que** le composé organosiliconé (ii) est choisi parmi les polysiloxanes de formule (I) ou les composés agissant comme agent d'extension de chaîne.

10. Composition selon la revendication précédente, **caractérisée par le fait que** le composé (ii) est un organohydrogenopolysiloxane liquide de formule (II) : 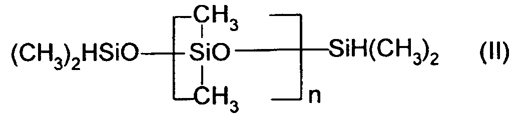 où n est un nombre entier supérieur à 1 et de préférence supérieur à 10.

11. Composition selon la revendication précédente, **caractérisée par le fait que**, dans la formule (II), n est égal à 20.

12. Composition selon l'une quelconque des revendications 4 à 11, **caractérisée par le fait que** la dispersion aqueuse de particules de copolymère siliconé est obtenue par mélange d'eau, d'au moins un émulsifiant, du polysiloxane (i), du composé organosiliconé (ii) et d'un catalyseur.

13. Composition selon la revendication précédente, **caractérisée par le fait que** la dispersion est une dispersion aqueuse de copolymère divinyldimethicone / dimethicone, C₁₂-C₁₃ Pareth-3 et C₁₂-C₁₃ Pareth-23.

14. Composition selon l'une quelconque des revendications précédentes, **caractérisée par le fait que** le monomère à insaturation éthylénique et à groupement sulfonique est choisi parmi l'acide vinylsulfonique, l'acide styrènesulfonique, les acides (méth)acrylamido(C₁-C₂₂)alkylsulfoniques, les acides N-(C₁-C₂₂)alkyl-(méth)acrylamido(C₁-C₂₂)alkylsulfoniques comme l'acide undécyl-acrylamido-méthane-sulfonique, ainsi que leurs formes partiellement ou totalement neutralisées, et leurs mélanges.

15. Composition selon l'une quelconque des revendications précédentes, **caractérisée par le fait que** le polymère comportant au moins un monomère à insaturation éthylénique et à groupement sulfonique est réticulé.

16. Composition selon la revendication précédente, **caractérisée par le fait que** l'agent de réticulation est choisi parmi le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou leurs mélanges.

17. Composition selon la revendication 15 ou 16, **caractérisée par le fait que** l'agent de réticulation est choisi parmi le méthylène-bis-acrylamide, le méthacrylate d'allyle ou le triméthylol propane triacrylate (TMPTA).

18. Composition selon l'une quelconque des revendications 15 à 17, **caractérisée par le fait que** le taux de réticulation va de 0,01 à 10% en mole par rapport au polymère.

19. Composition selon l'une quelconque des revendications précédentes, **caractérisée par le fait que** le polymère comportant au moins un monomère à insaturation éthylénique et à groupement sulfonique est un homopolymère réticulé et neutralisé d'acide 2-acrylamido 2-méthylpropane sulfonique.

20. Composition selon l'une quelconque des revendications précédentes, **caractérisée par le fait que** le polymère comportant au moins un monomère à insaturation éthylénique et à groupement sulfonique est un homopolymère amphiphile choisi parmi les polymères amphiphiles statistiques d'AMPS modifiés par réaction avec une n-monoalkylamine ou une di-n-alkylamine en C₆-C₂₂.

21. Composition selon l'une quelconque des revendications précédentes, **caractérisée par le fait que** le polymère comportant au moins un monomère à insaturation éthylénique et à groupement sulfonique est un copolymère obtenu à partir de monomères à insaturation éthylénique et à groupement sulfonique et de monomères à insaturation éthylénique sans groupement sulfonique.

22. Composition selon l'une quelconque des revendications précédentes, **caractérisée par le fait que** le polymère comportant au moins un monomère à insaturation éthylénique et à groupement sulfonique est un polymère amphiphile obtenu à partir d'AMPS et d'au moins un monomère hydrophobe à insaturation éthylénique comportant au moins une partie hydrophobe ayant de 6 à 50 atomes de carbone, de préférence de 6 à 22 atomes de carbone.

23. Composition selon la revendication précédente, **caractérisée par le fait que** le monomère hydrophobe à insaturation éthylénique est choisi parmi les acrylates ou les acrylamides de formule (III) suivante : 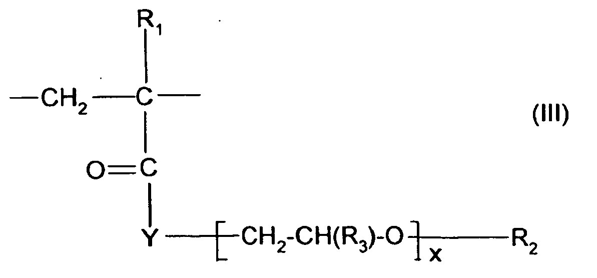 dans laquelle R₁ et R₃, identiques ou différents, désignent un atome d'hydrogène ou un radical alkyle substantiellement linéaire ou ramifié en C₁-C₆ (de préférence méthyle) ; Y désigne O ou NH ; R₂ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 0 à 100.

24. Composition selon la revendication précédente, **caractérisée par le fait que** le radical hydrophobe R₂ est choisi parmi les radicaux alkyles en C₆-C₁₈, substantiellement linéaires, ramifiés ou cycliques ; les radicaux alkylperfluorés en C₆-C₁₈ ; le radical cholestéryle ou un ester de cholestérol ; les groupes polycycliques aromatiques.

25. Composition selon la revendication 23 ou 24, **caractérisée par le fait que** le monomère de formule (III) comporte en plus au moins une chaîne polyoxyalkylénée.

26. Composition selon l'une quelconque des revendications 22 à 25, **caractérisée par le fait que** le polymère amphiphile est choisi parmi :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(meth)acrylate, par rapport au polymère ;
- les terpolymères comportant de 10 à 90% en mole de motifs acrylamide, de 0,1 à 10% en mole de motifs AMPS et de 5 à 80% en mole de motifs n-(C₆₋₁₈)alkylacrylamide, par rapport au polymère ;
- les copolymères non réticulés d'AMPS partiellement ou totalement neutralisé et de méthacrylate de n-dodécyle, de n-hexadécyle ou de n-octadécyle ;
- les copolymères réticulés ou non réticulés d'AMPS partiellement ou totalement neutralisé et de n-dodécylméthacrylamide.

27. Composition selon l'une quelconque des revendications 22 à 25, **caractérisée par le fait que** le polymère amphiphile est choisi parmi les copolymères constitués de motifs acide 2-acrylamido-2-méthylpropane-sulfonique (AMPS) de formule (IV) suivante : 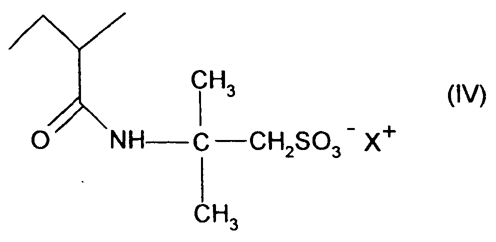 dans laquelle X⁺ est un proton, un cation de métal alcalin, un cation alcalino-terreux ou l'ion ammonium,
et de motifs de formule (V) suivante : 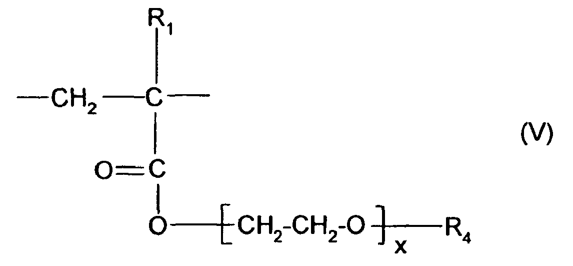 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle substantiellement linéaire ou ramifié en C₆-C₂₂ et plus préférentiellement en C₁₀-C₂₂.

28. Composition selon l'une quelconque des revendications précédentes, **caractérisée par le fait que** la quantité de polymère comportant au moins un monomère à insaturation éthylénique et à groupement sulfonique va de 0,01 à 20 % en poids, de préférence de 0,1 à 10 % en poids par rapport au poids total de la composition.

29. Composition selon l'une quelconque des revendications précédentes, **caractérisée par le fait que** la poudre organique est choisie parmi les particules de polyamide ; les poudres et billes de polyéthylène ; les microsphères à base de copolymères acryliques ou méthacryliques ; les microsphères de polyméthacrylate de méthyle ; les poudres de copolymère éthylène-acrylate ; les poudres expansées ; les poudres de matériaux organiques naturels ; les microbilles de résine de silicone ; et leurs mélanges.

30. Composition selon l'une quelconque des revendications précédentes, **caractérisée par le fait que** la poudre organique est présente en une quantité allant de 0,01 à 30 % en poids, et de préférence de 0,1 à 20 % en poids par rapport au poids total de la composition.

31. Composition selon l'une quelconque des revendications précédentes, **caractérisée par le fait qu'**elle comprend en outre un ou plusieurs adjuvants choisis parmi les agents gélifiants ; les agents hydratants ; les émollients ; les actifs ; les agents anti-radicaux libres ; les séquestrants ; les antioxydants ; les conservateurs ; les agents alcanisants ou acidifiants ; les parfums ; les agents filmogènes ; les matières colorantes ; les charges minérales ; et leurs mélanges.

32. Composition selon la revendication précédente, **caractérisée en ce que** l'adjuvant est un actif hydrophile sensible à l'oxydation choisi parmi l'acide ascorbique et ses dérivés tels que le 5,6-di-O-diméthylsilyl-ascorbate, le sel de potassium du dl-alpha-tocopheryl-2l-ascorbyl-phosphate, l'ascorbyl phosphate de magnésium, l'ascorbyl phosphate de sodium ; le phloroglucinol ; les enzymes ; et leurs mélanges.

33. Composition selon la revendication 31 ou 32, **caractérisée par le fait que** l'adjuvant est une charge minérale choisie parmi le talc ; le kaolin ; le nitrure de bore ; les oxydes métalliques ; les micas ; les nacres ; les poudres de silice ; l'oxychlorure de bismuth ; le stéarate de zinc ; les particules de sel de métal alcalin ou alcalinoterreux ; les particules de platine ; les alumines ; les alumino-silicates ; les silicates mixtes de métaux alcalins et/ou alcalino-terreux ; les zéolithes ; la magnésie ; les matériaux composites à base de charges minérales ; et leurs mélanges.

34. Composition selon la revendication précédente, **caractérisée par le fait que** la charge minérale est un oxyde métallique choisi parmi les oxydes de titane, de zinc, de fer, de zirconium, de cérium ou leurs mélanges.

35. Composition selon l'une quelconque des revendications précédentes, **caractérisée par le fait qu'**elle se présente sous forme d'une émulsion, et de préférence sous forme d'une émulsion H/E.

36. Composition selon l'une quelconque des revendications précédentes, **caractérisée par le fait qu'**elle constitue une composition cosmétique ou dermatologique.

37. Composition selon l'une quelconque des revendications précédentes, **caractérisée par le fait qu'**elle constitue un produit de soin, de protection, de nettoyage, de démaquillage et /ou de maquillage des matières kératiniques, un produit de protection solaire, un produit capillaire.

38. Composition selon la revendication précédente, **caractérisée par le fait que** la matière kératinique est la peau.

39. Utilisation cosmétique d'une composition cosmétique selon l'une quelconque des revendications 1 à 35, comme produit de soin, de nettoyage et/ou de démaquillage de la peau, des cheveux, du cuir chevelu, des cils, des sourcils, des ongles ou des muqueuses.

40. Utilisation cosmétique d'une composition cosmétique selon l'une quelconque des revendications 1 à 35, comme produit de maquillage.

41. Utilisation cosmétique d'une composition cosmétique selon l'une quelconque des revendications 1 à 35, comme produit de protection solaire.

42. Utilisation cosmétique d'une composition cosmétique selon l'une quelconque des revendications 1 à 35, comme produit capillaire rincé ou non-rincé.

43. Procédé de traitement cosmétique d'une matière kératinique, **caractérisé par le fait qu'**on applique sur la matière kératinique, une composition cosmétique telle que définie selon l'une quelconque des revendications 1 à 35.

## Claims

1. Composition comprising, in a physiologically acceptable medium, at least one aqueous phase comprising, in dispersion, particles of non-crosslinked block silicone copolymer obtained by chain extension, and either at least one polymer comprising at least one ethylenically unsaturated monomer containing a sulfonic group, or at least one organic powder, or a mixture of at least one polymer comprising at least one ethylenically unsaturated monomer containing a sulfonic group and of at least one organic powder.

2. Composition according to Claim 1, **characterized in that** the amount of silicone copolymer ranges from 0.01% to 15% by weight relative to the total weight of the composition.

3. Composition according to Claim 1 or 2, **characterized in that** the particles of silicone copolymer have a number-average size of less than or equal to 2 microns.

4. Composition according to any one of the preceding claims, **characterized in that** the silicone copolymer is obtained via a chain extension reaction, in the presence of a catalyst, from at least:
- (a) one polysiloxane (i) containing at least one reactive group and preferably one or two reactive groups per molecule; and
- (b) one organosilicone compound (ii) that reacts with the polysiloxane (i) via a chain extension reaction.

5. Composition according to the preceding claim, **characterized in that** the organopolysiloxane (i) is chosen from the compounds of formula (I): 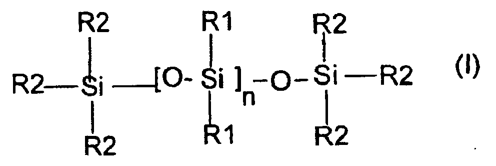 in which R₁ and R₂, independently of each other, represent a hydrocarbon-based group containing from 1 to 20 carbon atoms or an aryl group or a reactive group, and n is an integer greater than 1, provided that there are on average between one and two reactive groups per polymer.

6. Composition according to the preceding claim, **characterized in that** the reactive group is chosen from hydrogen; aliphatically unsaturated groups; a hydroxyl group; alkoxy groups; alkoxyalkoxy groups; an acetoxy group; amino groups; and mixtures thereof.

7. Composition according to Claim 5 or 6, **characterized in that** R₁ represents a methyl group.

8. Composition according to any one of Claims 5 to 7, **characterized in that** R₂ at the end of a chain represents a vinyl group.

9. Composition according to Claim 4, **characterized in that** the organosilicone compound (ii) is chosen from the polysiloxanes of formula (I) or compounds acting as chain extenders.

10. Composition according to the preceding claim, **characterized in that** compound (ii) is a liquid organohydrogenopolysiloxane of formula (II): 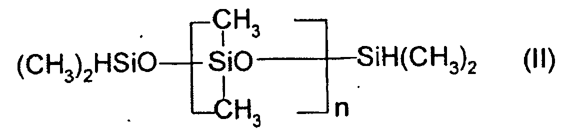 where n is an integer greater than 1 and preferably greater than 10.

11. Composition according to the preceding claim, **characterized in that**, in formula (II), n is equal to 20.

12. Composition according to any one of Claims 4 to 11, **characterized in that** the aqueous dispersion of particles of silicone copolymer is obtained by mixing water, at least one emulsifier, the polysiloxane (i), the organosilicone compound (ii) and a catalyst.

13. Composition according to the preceding claim, **characterized in that** the dispersion is an aqueous dispersion of divinyldimethicone/dimethicone copolymer, C₁₂-C₁₃ Pareth-3 and C₁₂-C₁₃ Pareth-23.

14. Composition according to any one of the preceding claims, **characterized in that** the ethylenically unsaturated monomer containing a sulfonic group is chosen from vinylsulfonic acid, styrenesulfonic acid, (meth)acrylamido(C₁-C₂₂)alkylsulfonic acids, N-(C₁-C₂₂)alkyl(meth)acrylamido(C₁-C₂₂)alkylsulfonic acids, for instance undecylacrylamidomethanesulfonic acid, and also the partially or totally neutralized forms thereof, and mixtures thereof.

15. Composition according to any one of the preceding claims, **characterized in that** the polymer comprising at least one ethylenically unsaturated monomer containing a sulfonic group is crosslinked.

16. Composition according to the preceding claim, **characterized in that** the crosslinking agent is chosen from divinylbenzene, diallyl ether, dipropylene glycol diallyl ether, polyglycol diallyl ethers, triethylene glycol divinyl ether, hydroquinone diallyl ether, ethylene glycol or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allylic or vinyl ethers of polyfunctional alcohols, and also the allylic esters of phosphoric and/or vinylphosphonic acid derivatives, or mixtures thereof.

17. Composition according to Claim 15 or 16, **characterized in that** the crosslinking agent is chosen from methylenebisacrylamide, allyl methacrylate and trimethylolpropane triacrylate (TMPTA).

18. Composition according to any one of Claims 15 to 17, **characterized in that** the degree of crosslinking ranges from 0.01 mol% to 10 mol% relative to the polymer.

19. Composition according to any one of the preceding claims, **characterized in that** the polymer comprising at least one ethylenically unsaturated monomer containing a sulfonic group is a crosslinked and neutralized 2-acrylamido-2-methylpropanesulfonic acid homopolymer.

20. Composition according to any one of the preceding claims, **characterized in that** the polymer comprising at least one ethylenically unsaturated monomer containing a sulfonic group is an amphiphilic homopolymer chosen from random amphiphilic polymers of AMPS modified by reaction with a C₆-C₂₂ mono-n-alkylamine or di-n-alkylamine.

21. Composition according to any one of the preceding claims, **characterized in that** the polymer comprising at least one ethylenically unsaturated monomer containing a sulfonic group is a copolymer obtained from ethylenically unsaturated monomers containing a sulfonic group and ethylenically unsaturated monomers not containing a sulfonic group.

22. Composition according to any one of the preceding claims, **characterized in that** the polymer comprising at least one ethylenically unsaturated monomer containing a sulfonic group is an amphiphilic polymer obtained from AMPS and from at least one ethylenically unsaturated hydrophobic monomer comprising at least one hydrophobic portion containing from 6 to 50 carbon atoms and preferably from 6 to 22 carbon atoms.

23. Composition according to the preceding claim, **characterized in that** the ethylenically unsaturated hydrophobic monomer is chosen from the acrylates and the acrylamides of formula (III) below: 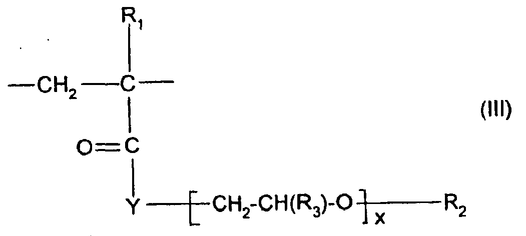 in which R₁ and R₃, which may be identical or different, denote a hydrogen atom or a substantially linear or branched C₁-C₆ alkyl radical (preferably methyl); Y denotes O or NH; R₂ denotes a hydrophobic hydrocarbon-based radical containing at least from 6 to 50 carbon atoms, more preferably from 6 to 22 carbon atoms, even more preferably from 6 to 18 carbon atoms and more particularly from 12 to 18 carbon atoms; and x denotes a number of moles of alkylene oxide and ranges from 0 to 100.

24. Composition according to the preceding claim, **characterized in that** the hydrophobic radical R₂ is chosen from substantially linear, branched or cyclic C₆-C₁₈ alkyl radicals; C₆-C₁₈ perfluoroalkyl radicals; the cholesteryl radical or a cholesterol ester; and aromatic polycyclic groups.

25. Composition according to Claim 23 or 24, **characterized in that** the monomer of formula (III) also comprises at least one polyoxyalkylenated chain.

26. Composition according to any one of Claims 22 to 25, **characterized in that** the amphiphilic polymer is chosen from:
- crosslinked or non-crosslinked and neutralized or non-neutralized copolymers comprising from 15% to 60% by weight of AMPS units and from 40% to 85% by weight of (C₈-C₁₆)alkyl(meth)acrylamide units or (C₈-C₁₆)alkyl (meth)acrylate units, relative to the polymer;
- terpolymers comprising from 10 mol% to 90 mol% of acrylamide units, from 0.1 mol% to 10 mol% of AMPS units and from 5 mol% to 80 mol% of n-(C₆-C₁₈)alkyl-acrylamide units, relative to the polymer;
- non-crosslinked copolymers of partially or totally neutralized AMPS and of n-dodecyl, n-hexadecyl or n-octadecyl methacrylate; and
- crosslinked or non-crosslinked copolymers of partially or totally neutralized AMPS and of n-dodecylmethacrylamide.

27. Composition according to any one of Claims 22 to 25, **characterized in that** the amphiphilic polymer is chosen from copolymers consisting of 2-acrylamido-2-methylpropanesulfonic acid (AMPS) units of formula (IV) below: 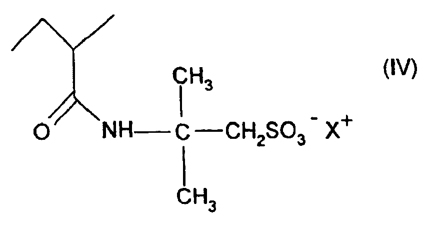 in which X⁺ is a proton, an alkali metal cation, an alkaline-earth metal cation or an ammonium ion,
and of units of formula (V) below: 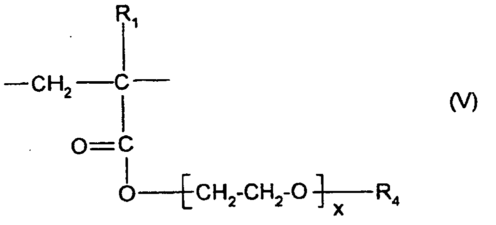 in which x denotes an integer ranging from 3 to 100, preferably from 5 to 80 and more preferably from 7 to 25; R₁ has the same meaning as that indicated above in formula (I) and R₄ denotes a substantially linear or branched C₆-C₂₂ and more preferably C₁₀-C₂₂ alkyl.

28. Composition according to any one of the preceding claims, **characterized in that** the amount of polymer comprising at least one ethylenically unsaturated monomer containing a sulfonic group ranges from 0.01% to 20% by weight and preferably from 0.1% to 10% by weight relative to the total weight of the composition.

29. Composition according to any one of the preceding claims, **characterized in that** the organic powder is chosen from polyamide particles; polyethylene powders and beads; microspheres based on acrylic or methacrylic copolymers; polymethyl methacrylate microspheres; ethylene-acrylate copolymer powders; expanded powders; powders of natural organic materials; silicone resin microbeads; and mixtures thereof.

30. Composition according to any one of the preceding claims, **characterized in that** the organic powder is present in an amount ranging from 0.01% to 30% by weight and preferably from 0.1% to 20% by weight relative to the total weight of the composition.

31. Composition according to any one of the preceding claims, **characterized in that** it also comprises one or more adjuvants chosen from gelling agents; moisturizers; emollients; active agents; free-radical scavengers; sequestrants; antioxidants; preserving agents; acidifying or basifying agents; fragrances; film-forming agents; dyestuffs; mineral fillers; and mixtures thereof.

32. Composition according to the preceding claim, **characterized in that** the adjuvant is an oxidation-sensitive hydrophilic active agent chosen from ascorbic acid and its derivatives such as 5,6-di-O-dimethylsilyl ascorbate, the potassium salt of dl-α-tocopheryl-2l-ascorbyl phosphate, magnesium ascorbyl phosphate and sodium ascorbyl phosphate; phloroglucinol; enzymes; and mixtures thereof.

33. Composition according to Claim 31 or 32, **characterized in that** the adjuvant is a mineral filler chosen from talc; kaolin; boron nitride; metal oxides; micas; nacres; silica powders; bismuth oxychloride; zinc stearate; particles of alkali metal or alkaline-earth metal salts; platinum particles; aluminas; aluminosilicates; mixed silicates of alkali metals and/or alkaline-earth metals; zeolites; magnesia; composite materials based on mineral fillers; and mixtures thereof.

34. Composition according to the preceding claim, **characterized in that** the mineral filler is a metal oxide chosen from titanium oxide, zinc oxide, iron oxide, zirconium oxide and cerium oxide, or mixtures thereof.

35. Composition according to any one of the preceding claims, **characterized in that** it is in the form of an emulsion and preferably in the form of an O/W emulsion.

36. Composition according to any one of the preceding claims, **characterized in that** it constitutes a cosmetic or dermatological composition.

37. Composition according to any one of the preceding claims, **characterized in that** it constitutes a care, protective, cleansing, makeup and/or makeup-removing product for keratin materials, an antisun product or a haircare product.

38. Composition according to the preceding claim, **characterized in that** the keratin material is the skin.

39. Cosmetic use of a cosmetic composition according to any one of Claims 1 to 35, as a care, cleansing and/or makeup-removing product for the skin, the hair, the scalp, the eyelashes, the eyebrows, the nails or mucous membranes.

40. Cosmetic use of a cosmetic composition according to any one of Claims 1 to 35, as a makeup product.

41. Cosmetic use of a cosmetic composition according to any one of Claims 1 to 35, as an antisun product.

42. Cosmetic use of a cosmetic composition according to any one of Claims 1 to 35, as a rinse-out or leave-in haircare product.

43. Cosmetic process for treating a keratin material, **characterized in that** a cosmetic composition as defined according to any one of Claims 1 to 35 is applied to the keratin material.

## Patentansprüche

1. Zusammensetzung, die in einem physiologisch akzeptablen Medium mindestens eine wässerige Phase enthält, die in Dispersion Partikel eines nicht vernetzten, durch Kettenverlängerung erhaltenen Silicon-Blockcopolymers und entweder mindestens ein Polymer, das mindestens ein Monomer mit ethylenischer Doppelbindung und Sulfonsäuregruppe umfasst, oder mindestens ein organisches Pulver oder ein Gemisch aus mindestens einem Polymer, das mindestens ein Monomer mit ethylenischer Doppelbindung und Sulfonsäuregruppe umfasst, und mindestens einem organischen Pulver enthält.

2. Zusammensetzung nach Anspruch 1, **dadurch gekennzeichnet, dass** der Mengenanteil des Silicon-Copolymers im Bereich von 0,01 bis 15 Gew.-%, bezogen auf das Gesamtgewicht der Zusammensetzung, liegt.

3. Zusammensetzung nach Anspruch 1 oder 2, **dadurch gekennzeichnet, dass** die Partikel des Silicon-Copolymers eine zahlenmittlere Größe von 2 Mikrometer oder darunter aufweisen.

4. Zusammensetzung nach einem der vorhergehenden Ansprüche, **dadurch gekennzeichnet, dass** das Silicon-Copolymer durch Kettenverlängerung in Gegenwart eines Katalysators ausgehend von zumindest den folgenden Verbindungen hergestellt wird:
- (a) eines Polysiloxans (i) mit mindestens einer reaktiven Gruppe und vorzugsweise einer oder zwei reaktiven Gruppen pro Molekül und
- (b) einer Silicium-organischen Verbindung (ii), die mit dem Polysiloxans (i) unter Kettenverlängerung reagiert.

5. Zusammensetzung nach dem vorhergehenden Anspruch, **dadurch gekennzeichnet, dass** das Organopolysiloxan (i) unter den Verbindungen der folgenden Formel (I) ausgewählt ist, 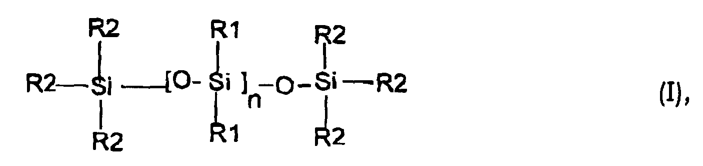 worin die Gruppen R₁ und R₂ unabhängig voneinander eine Kohlenwasserstoffgruppe mit 1 bis 20 Kohlenstoffatomen oder eine Arylgruppe oder eine reaktive Gruppe bedeuten und n eine ganze Zahl über 1 ist, mit der Maßgabe, dass im Mittel eine bis zwei reaktive Gruppen pro Polymer vorhanden sind.

6. Zusammensetzung nach dem vorhergehenden Anspruch, **dadurch gekennzeichnet, dass** die reaktive Gruppe unter Wasserstoff, aliphatisch ungesättigten Gruppen; der Hydroxygruppe; Alkoxygruppen; Alkoxy-alkoxy-Gruppen; der Acetoxygruppe; aminierten Gruppen; und deren Gemischen ausgewählt ist.

7. Zusammensetzung nach Anspruch 5 oder 6, **dadurch gekennzeichnet, dass** die Gruppe R₁ Methyl bedeutet.

8. Zusammensetzung nach Anspruch 5 bis 7, **dadurch gekennzeichnet, dass** die Gruppe R₂ am Ende der Kette eine Vinylgruppe bedeutet.

9. Zusammensetzung nach Anspruch 4, **dadurch gekennzeichnet, dass** die Silicium-organische Gruppe (ii) unter den Polysiloxanen der Formel (I) ausgewählt ist, wenn die Verbindungen als Kettenverlängerer wirken.

10. Zusammensetzung nach dem vorhergehenden Anspruch, **dadurch gekennzeichnet, dass** die Verbindung (ii) ein flüssiges Organohydrogenopolysiloxan der Formel (II) ist: 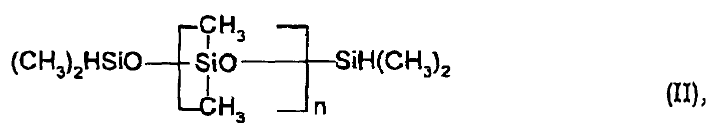 worin n eine ganze Zahl über 1 und vorzugsweise über 10 bedeutet.

11. Zusammensetzung nach dem vorhergehenden Anspruch, **dadurch gekennzeichnet, dass** in der Formel (II) n über 20 liegt.

12. Zusammensetzung nach einem der Ansprüche 4 bis 11, **dadurch gekennzeichnet, dass** die wässerige Dispersion der Partikel des Silicon-Copolymers durch Mischen von Wasser, mindestens eines Emulgators, des Polysiloxans (i), der Silicium-organischen Verbindung (ii) und eines Katalysators hergestellt wird.

13. Zusammensetzung nach dem vorhergehenden Anspruch, **dadurch gekennzeichnet, dass** die Dispersion eine wässerige Dispersion aus Divinyldimethicon/Dimethicon-Copolymer; C₁₂-C₁₃₋Pareth-3 und C₁₂-C₁₃ Pareth-23 ist.

14. Zusammensetzung nach einem der vorhergehenden Ansprüche, **dadurch gekennzeichnet, dass** das Monomer mit ethylenischer Doppelbindung und Sulfonsäuregruppe unter Vinylsulfonsäure, Styrolsulfonsäure, (Meth)acrylamido(C₁₋₂₂)alkylsulfonsäuren, N-(C₁₋₂₂)Alkyl(meth)acrylamido(C₁₋₂₂)alkylsulfonsäuren, wie Undecyl-acrylamido-methansulfonsäure sowie ihren ganz oder teilweise neutralisierten Formen und deren Gemischen ausgewählt ist.

15. Zusammensetzung nach einem der vorhergehenden Ansprüche, **dadurch gekennzeichnet, dass** das Polymer, das mindestens ein Monomer mit ethylenischer Doppelbindung und Sulfonsäuregruppe enthält, vernetzt ist.

16. Zusammensetzung nach dem vorhergehenden Anspruch, **dadurch gekennzeichnet, dass** das Vernetzungsmittel unter Divinylbenzol, Diallylether, Dipropylenglykol-diallylether, Polyglykoldiallylethern, Triethylenglykol-divinylether, Hydrochinondiallylether, Ethylenglykol-di(meth)acrylat oder Tetraethylenglykol-di(meth)acrylat, Trimethylolpropantriacrylat, Methylen-bisacrylamid, Methylen-bis-methacrylamid, Triallylamin, Triallylcyanurat, Diallylmaleat, Tetraallylethylendiamin, Tetraallyloxyethan, Trimethylolpropan-diallylether, Allyl(meth)acrylat, Allylethern von Alkoholen aus der Zuckergruppe oder anderen Allyl- oder Vinylethern von polyfunktionellen Alkoholen sowie Allylestern von Phosphorsäurederivaten und/oder Vinylphosphonsäurederivaten oder deren Gemischen ausgewählt ist.

17. Zusammensetzung nach Anspruch 15 oder 16, **dadurch gekennzeichnet, dass** das Vernetzungsmittel unter Methylen-bisacrylamid, Allylmethacrylat oder Trimethylolpropantriacrylat (TMPTA) ausgewählt ist.

18. Zusammensetzung nach einem der Ansprüche 15 bis 17, **dadurch gekennzeichnet, dass** der Vernetzungsgrad im Bereich von 0,01 bis 10 Mol-%, bezogen auf das Polymer, beträgt.

19. Zusammensetzung nach einem der vorhergehenden Ansprüche, **dadurch gekennzeichnet, dass** das Polymer mit mindestens einem Monomer mit ethylenischer Doppelbindung und Sulfonsäuregruppe ein vemetztes und neutralisiertes 2-Acrylamido-2-methylpropansulfonsäure-Homopolymer ist.

20. Zusammensetzung nach einem der vorhergehenden Ansprüche, **dadurch gekennzeichnet, dass** das Polymer, das mindestens ein Monomer mit ethylenischer Doppelbindung und Sulfonsäuregruppe umfasst, ein amphiphiles Homopolymer ist, das unter den statistischen amphiphilen Polymeren von AMPS ausgewählt ist, die durch Umsetzung mit einem *n*-Monoalkylamin oder einem Di-*n*-alkylamin mit 6 bis 22 Kohlenstoffatomen modifiziert wurden.

21. Zusammensetzung nach einem der vorhergehenden Ansprüche, **dadurch gekennzeichnet, dass** das Polymer, das mindestens ein Monomer mit ethylenischer Doppelbindung und Sulfonsäuregruppe umfasst, ein Copolymer ist, das ausgehend von Monomeren mit ethylenischer Doppelbindung und Sulfonsäuregruppe und Monomeren mit ethylenischer Doppelbindung ohne Sulfonsäuregruppe hergestellt wurde.

22. Zusammensetzung nach einem der vorhergehenden Ansprüche, **dadurch gekennzeichnet, dass** das Polymer, das mindestens ein Monomer mit ethylenischer Doppelbindung und Sulfonsäuregruppe umfasst, ein amphiphiles Polymer ist, das, ausgehend von AMPS und mindestens einem hydrophoben Monomer mit ethylenischer Doppelbindung hergestellt ist, welches mindestens einen hydrophoben Teil mit 6 bis 50 Kohlenstoffatomen und vorzugsweise 6 bis 22 Kohlenstoffatomen aufweist.

23. Zusammensetzung nach dem vorhergehenden Anspruch, **dadurch gekennzeichnet, dass** das hydrophobe Monomer mit ethylenischer Doppelbindung unter den Acrylaten oder Acrylamiden der folgenden Formel (III) ausgewählt ist: 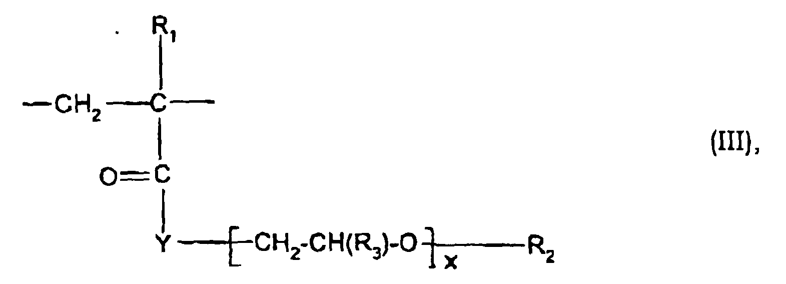 worin die Gruppen R₁ und R₃, die gleich oder verschieden sind, ein Wasserstoffatom oder eine im Wesentlichen geradkettige oder verzweigte Alkylgruppe mit 1 bis 6 Kohlenstoffatomen und vorzugsweise Methyl bedeuten; Y O oder NH bedeutet; R₂ eine hydrophobe Kohlenwasserstoffgruppe mit mindestens 6 bis 50 Kohlenstoffatomen und vorzugsweise 6 bis 22 Kohlenstoffatomen oder noch bevorzugter 6 bis 18 Kohlenstoffatomen und insbesondere 12 bis 18 Kohlenstoffatome bedeutet; x, dessen Wert die Anzahl der Mol Alkylenoxid angibt, im Bereich von 0 bis 100 liegt.

24. Zusammensetzung nach dem vorhergehenden Anspruch, **dadurch gekennzeichnet, dass** die hydrophobe Gruppe R₂ unter den in etwa geradkettigen, verzweigten oder cyclischen C₆-₁₈₋Alkylgruppen; alkylperfluorierten Gruppen mit 6 bis 18 Kohlenstoffatomen; der Cholesterylgruppe oder einer Cholesterinestergruppe; und aromatischen polycyclischen Gruppen ausgewählt ist.

25. Zusammensetzung nach Anspruch 23 oder 24, **dadurch gekennzeichnet, dass** das Monomer der Formel (III) ferner mindestens eine polyalkoxylierte Kette aufweist.

26. Zusammensetzung nach einem der Ansprüche 22 bis 25, **dadurch gekennzeichnet, dass** das amphiphile Polymer ausgewählt ist unter:
- vernetzten oder nicht vernetzten, neutralisierten oder nicht neutralisierten Copolymeren mit 15 bis 60 Gew.-% AMPS-Einheiten und 40 bis 85 Gew.-% C₈₋₁₆-Alkyl(meth)acrylamideinheiten oder C₈₋₁₅-Alkyl(meth)acrylateinheiten, bezogen auf das Polymer;
- Terpolymeren mit 10 bis 90 Mol-% Acrylamideinheiten, 0,1 bis 10 Mol-% AMPS-Einheiten und 5 bis 80 Mol-% *n*-(C₆₋₁₈)-Alkylacrylamideinheiten, bezogen auf das Polymer;
- nicht vernetzten Copolymeren von ganz oder teilweise neutralisierter AMPS und *n*-Dodecylmethacrylat, *n*-Hexadecylmethacrylat oder *n*-Octadecylmethacrylat;
- vernetzten oder nicht vernetzten Copolymeren von ganz oder teilweise neutralisiertem AMPS und *n*-Dodecylmethacrylamid.

27. Zusammensetzung nach einem der Ansprüche 22 bis 25, **dadurch gekennzeichnet, dass** das amphiphile Polymer unter den Copolymeren ausgewählt ist, die aus 2-Acrylamido-2-methylpropansulfonsäure (AMPS)-Einheiten der folgenden Formel (IV): 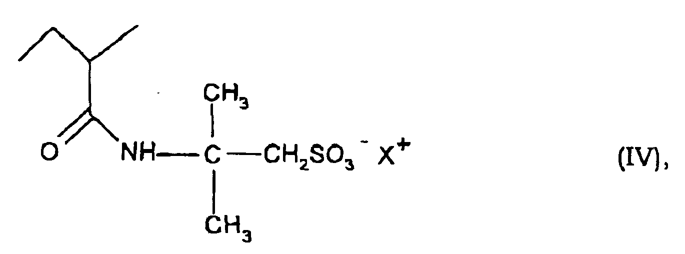 worin X⁺ ein Proton, ein Alkalimetallkation, ein Erdalkalimetallkation oder Ammonium bedeutet, und Einheiten der folgenden Formel (V) bestehen: 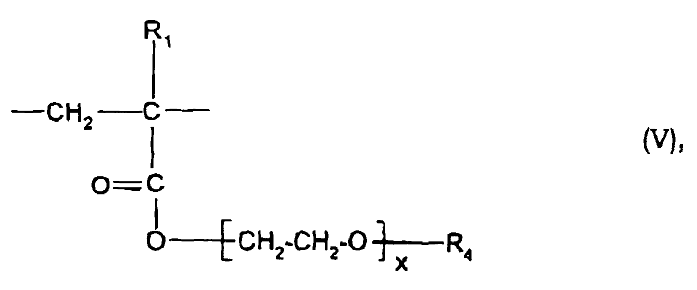 worin x eine ganze Zahl von 3 bis 100 und vorzugsweise 5 bis 80 und noch bevorzugter 7 bis 25 bedeutet; R₁ die oben für die Formel (I) angegebenen Bedeutungen aufweist und R₄ eine in etwa lineare oder verzweigte Alkylgruppe mit 6 bis 22 und vorzugsweise 10 bis 22 Kohlenstoffatomen bedeutet.

28. Zusammensetzung nach einem der vorhergehenden Ansprüche, **dadurch gekennzeichnet, dass** der Mengenanteil des Polymers mit mindestens einem Monomer mit ethylenischer Doppelbindung und Sulfonsäuregruppe im Bereich von 0,01 bis 20 Gew.-% und vorzugsweise 0,1 bis 10 Gew,-%, bezogen auf das Gesamtgewicht der Zusammensetzung, liegt.

29. Zusammensetzung nach einem der vorhergehenden Ansprüche, **dadurch gekennzeichnet, dass** das organische Pulver unter den Partikeln von Polyamid; Polyethylenpulvern und Polyethylenkügelchen; Mikrosphären auf der Basis von Acrylcopolymeren oder Methacrylcopolymeren; Mikrosphären von Polymethylmethacrylat; Pulvern aus Ethylen/Acrylat-Copolymer; expandierten Pulvern; Pulvern von natürlichen organischen Materialien, Siliconharzmikrokugeln und deren Gemischen ausgewählt ist.

30. Zusammensetzung nach einem der vorhergehenden Ansprüche, **dadurch gekennzeichnet, dass** das organische Pulver in einer Menge von 0,01 bis 30 Gew.-% und vorzugsweise 0,1 bis 20 Gew.-%, bezogen auf das Gesamtgewicht der Zusammensetzung, enthalten ist.

31. Zusammensetzung nach einem der vorhergehenden Ansprüche, **dadurch gekennzeichnet, dass** sie ferner einen oder mehrere Zusatzstoffe enthält, die unter den Gelbildnern; Hydratisierungsmitteln; Emollientien; Wirkstoffen; Radikalfängern für freie Radikale; Maskierungsmitteln; Antioxidantien; Konservierungsmitteln; Alkalisierungsmitteln oder Ansäuerungsmitteln; Parfums; Filmbildnern; Farbmitteln; mineralischen Füllstoffen; und deren Gemischen ausgewählt ist.

32. Zusammensetzung nach dem vorhergehenden Anspruch, **dadurch gekennzeichnet, dass** der Zusatzstoff ein oxidationsempfindlicher hydrophiler Wirkstoff ist, der unter Ascorbinsäure und ihren Derivaten, wie 5,6-Di-O-dimethylsilyl-ascorbat, dem Kaliumsalz von dl-alpha-tocopheryl-21-ascorbylphosphat, Magnesiumascorbylphosphat, Natriumascorbylphosphat; Phloroglucin; Enzymen; und deren Gemischen ausgewählt ist.

33. Zusammensetzung nach Anspruch 31 oder 32, **dadurch gekennzeichnet, dass** der Zusatzstoff ein anorganischer Füllstoff ist, der unter Talk; Kaolin; Bornitrid; Metalloxiden; Glimmern; Perlglanzpigmenten; Siliciumdioxidpulvern; Bismutoxidchlorid; Zinkstearat; Partikeln von Alkali- oder Erdalkalisalzen; Platinpartikeln; Aluminiumoxiden; Aluminosilicaten; gemischten Silicaten von Alkalimetallen und/oder Erdalkalimetallen; Zeolithen; Magnesiumoxid; zusammengesetzten Materialien auf der Basis von anorganischen Füllstoffen; und deren Gemischen ausgewählt ist.

34. Zusammensetzung nach dem vorhergehenden Anspruch, **dadurch gekennzeichnet, dass** der anorganische Füllstoff ein Metalloxid ist, das unter den Oxiden von Titan, Zink, Eisen, Zirconium, Cer oder deren Gemischen ausgewählt ist.

35. Zusammensetzung nach einem der vorhergehenden Ansprüche, **dadurch gekennzeichnet, dass** sie in Form einer Emulsion und vorzugsweise als O/W-Emulsion vorliegt.

36. Zusammensetzung nach einem der vorhergehenden Ansprüche, **dadurch gekennzeichnet, dass** es sich um eine kosmetische oder dermatologische Zusammensetzung handelt.

37. Zusammensetzung nach einem der vorhergehenden Ansprüche, **dadurch gekennzeichnet, dass** es sich um ein Produkt zur Pflege, zum Schutz, zur Reinigung, zum Abschminken und/oder zum Schminken von Keratinsubstanzen, Produkte zum Sonnenschutz und Produkte für die Haarbehandlung handelt.

38. Zusammensetzung nach dem vorhergehenden Anspruch, **dadurch gekennzeichnet, dass** die Keratinsubstanz die Haut ist.

39. Kosmetische Verwendung einer kosmetischen Zusammensetzung nach einem der Ansprüche 1 bis 35 als Produkt zur Pflege, zur Reinigung und/oder zum Abschminken der Haut, der Haare, der Kopfhaut, der Wimpern, der Augenbrauen, der Nägel oder der Schleimhäute.

40. Kosmetische Verwendung einer kosmetischen Zusammensetzung nach einem der Ansprüche 1 bis 35 als Produkt zum Schminken.

41. Kosmetische Verwendung einer kosmetischen Zusammensetzung nach einem der Ansprüche 1 bis 35 als Produkt zum Sonnenschutz.

42. Kosmetische Verwendung einer kosmetischen Zusammensetzung nach einem der Ansprüche 1 bis 35 als Produkt für die Haarbehandlung, das ausgespült wird oder im Haar verbleibt.

43. Verfahren zur kosmetischen Behandlung einer Keratinsubstanz, **dadurch gekennzeichnet, dass** auf die Keratinsubstanz eine kosmetische Zusammensetzung nach einem der Ansprüche 1 bis 35 aufgetragen wird.
